(19) Europäisches Patentamt / European Patent Office / Office européen des brevets

(11) **EP 1 782 774 A1**

(12) **EUROPEAN PATENT APPLICATION**
published in accordance with Art. 158(3) EPC

(43) Date of publication:
**09.05.2007 Bulletin 2007/19**

(51) Int Cl.:
**A61F 7/08** (2006.01)    **C09K 5/16** (2006.01)

(21) Application number: **05765767.8**

(22) Date of filing: **14.07.2005**

(86) International application number:
**PCT/JP2005/013003**

(87) International publication number:
**WO 2006/006650 (19.01.2006 Gazette 2006/03)**

(84) Designated Contracting States:
**DE FR GB**

(30) Priority: **14.07.2004 JP 2004207831**

(71) Applicant: **Mycoal Products Corporation**
**Tochigi-chi, Tochigi 328-0067 (JP)**

(72) Inventor: **DODO, Toshihiro,**
**c/o MYCOAL PRODUCTS CORPORATION**
**Tochigi-shi,**
**Tochigi 328-0067 (JP)**

(74) Representative: **Thun, Clemens et al**
**Mitscherlich & Partner**
**Sonnenstrasse 33**
**80331 München (DE)**

(54)    **HEATING ELEMENT AND PROCESS FOR PRODUCING HEATING ELEMENT**

(57)    There is provided a heat generating body which by forming a temporary adhering part upon temporary adhesion between a substrate and a heat generating composition molded body and a covering material with an adhesive layer and subsequent heat sealing, realizes higher line speed, thereby largely improving the productivity, does not generate position deviation of the heat generating composition molded body in welding the substrate and the covering material, is able to be heat sealed and is free from seal failure.

A heat generating body as formed by laminating a heat generating composition molded body resulting from molding of a heat generating composition containing, as essential components, an exothermic substance, a carbon component, a reaction accelerator and water and having a water mobility value of from 0.01 to 20 on a substrate, covering by a covering material and heat sealing the periphery of the heat generating composition molded body, is **characterized in that** the substrate and the covering material are temporarily adhered with a sticky layer, the temporarily adhered portion is heat sealed with a heat seal layer which the substrate and/or the covering material has, the sticky layer component and the heat seal layer component are co-present in the heat seal part, and the heat seal part has a seal strength at 60°C of 0.8 kg/25 mm or more.

*FIG. 2*

**Description**

[Technical Field]

The present invention relates to a heat generating body which upon temporary adhesion of a substrate and a covering material with an adhesive layer and subsequent heat sealing, is able to be surely heat sealed without generating wrinkles, realizes higher line speed, thereby largely improving the productivity and is free from seal failure and to a process for producing the same.

[Background Art]

In recent years, there have been proposed heat generating bodies having a heat generating composition sealed in a bag body in which at least one surface thereof is made of a air-permeable film (or a sheet) and thermal wet packs in which an adhesive layer is laminated on one surface of a heat generating body, thereby enabling to be stuck to a human body or the like. As so-called throwaway body warmers and thermal wet packs, there have also been proposed ones utilizing a warming measure or a medical hot pack and ones utilizing a so-called percutaneous absorption system in which an adhesive layer of such a thermal wet pack or a sticking agent contains or supports a percutaneously absorptive drug.

As a process for producing such a heat generating body, in general, a heat generating composition is thrown down in a prescribed region on the upper surface of an air-permeable or airtight substrate, an airtight or air-permeable covering material is then covered thereon, and thereafter, the periphery of the substrate and the periphery of the covering material are entirely heat sealed. Namely, the peripheries of the substrate and the covering material are entirely heat sealed.

In this case, there may be the case where a hot melt based bonding agent layer is laminated on either one of the substrate or the covering material and this hot melt based bonding agent layer is mediated.

Furthermore, as another process for producing such a heat generating body, in general, a heat generating composition is thrown down in a prescribed region on the upper surface of an air-permeable or airtight substrate, an airtight or air-permeable covering material is then covered thereon, and thereafter, the outer periphery of the substrate and the outer periphery of the covering material are entirely compression sealed via a sticky layer. Namely, the outer peripheries of the substrate and the covering material are entirely compression sealed (sealed with an adhesive).

Then, for the purpose of inhibiting an exothermic reaction until the time of use, the thus produced heat generating body is sealed and stored within an airtight outer bag and then provided for distribution.

In these conventional heat generating bodies, in the case where the outer peripheries of the substrate and the covering material are entirely heat sealed, there are encountered the following problems.

That is, in the case where by using a synthetic resin film which constitutes the substrate or the covering material or a hot melt based bonding agent layer which is formed in advance on at least one surface of the substrate or the covering material, the surroundings of the substrate and the covering material are heat sealed by heat fusion or heat bonding, for the purpose of preventing the heat generating composition from incorporation into the heat seal part, it is required to surely achieve heat fusion or heat bonding after melting the foregoing synthetic resin film or hot melt based bonding agent. Therefore, a limit for realizing high speed is generated, resulting in a lowering of the productivity.

Furthermore, in the case where a heat generating composition molded body is laminated on a substrate, a covering material is covered thereon, and the surroundings of the heat generating composition molded boy are heat sealed by heat fusion or heat bonding, there is some possibility that the heat generating composition molded body moves between the substrate and the covering material, thereby causing seal failure. In particular, such becomes remarkable at the time of high speed. Thus, even when lamination could have been achieved at a high speed, sealing could be achieved only at a low speed. As a result, it had to accept low-speed production as a whole of the step. In particular, in the case the volume of the heat generating composition molded body is small and a seal width is small, such became especially remarkable, resulting in an extra-low speed.

On the other hand, in an example where the surroundings of a substrate and the periphery of a covering material are adhesive sealed by using an adhesive layer, sealing can be simply achieved, and a heat generating body in which the seal portion, namely the entire surroundings of the heat generating body are flexible can be prepared. However, according to the foregoing adhesive sealing, a seal strength is lowered depending upon the utility at the time of using the heat generating body, the heat generation due to seal separation becomes instable so that there is created an anxiety such a burn. Thus, its use and utility were limited. Even when a temperature lower than the melting point of the adhesive layer is partially applied to achieve warm treatment and sealing, there was encountered a problem such that its effect is not so high, seal separation occurs and seal failure is caused, thereby causing abnormal heat generation.

As a matter of course, there are encountered exactly the same problems in the sticking agent to be used in this conventional heat generating body.

[Disclosure of the Invention]

[Problems that the Invention is to Solve]

An object of the invention is to solve the foregoing technical problems and to provide a heat generating body which by forming a temporary adhering part upon temporary adhesion between a substrate and a heat generating composition molded body and a covering material with an adhesive layer and subsequent heat sealing, realizes higher line speed, thereby largely improving the productivity, does not generate position deviation of the heat generating composition molded body in welding the substrate and the covering material, is able to be heat sealed and is free from seal failure.

[Means for Solving the Problems]

As set forth in claim 1, a heat generating body of the invention is a heat generating body as formed by laminating a heat generating compositionmoldedbody resulting frommolding of a heat generating composition containing, as essential components, an exothermic substance, a carbon component, a reaction accelerator and water and having a water mobility value of from 0.01 to 20 on a substrate, covering by a covering material and heat sealing the periphery of the heat generating composition molded body, which is characterized in that:

the substrate and the covering material are temporarily adhered with a sticky layer, the temporarily adhered portion is heat sealed with a heat seal layer which the substrate and/or the covering material has, the sticky layer component and the heat seal layer component are co-present in the heat seal part, and the heat seal part has a seal strength at 60°C of 0.8 kg/25 mm or more.
Also, a heat generating body as set forth in claim 2 is characterized in that in the heat generating body as set forth in claim 1, at least a part of the heat seal part has a region having an air vent part heat sealed therein.
Also, a heat generating body as set forth in claim 3 is characterized in that in the heat generating body as set forth in claim 1, the sticky layer is constituted of a hot melt based adhesive.
Also, a heat generating body as set forth in claim 4 is characterized in that in the heat generating body as set forth in claim 1, the total thickness of the heat seal layer is the thickness of the sticky layer or more.
Also, a heat generating body as set forth in claim 5 is characterized in that in the heat generating body as set forth in claim 1, the sticky layer has a thickness of from 0.1 to 100 $\mu$m.
Also, a heat generating body as set forth in claim 6 is characterized in that in the heat generating body as set forth in claim 1, the sticky layer has a void.
Also, a heat generating body as set forth in claim 7 is characterized in that in the heat generating body as set forth in claim 1, a mixed part of the heat seal layer component and the adhesive layer component is present in the heat seal part.
Also, a heat generating body as set forth in claim 8 is characterized in that in the heat generating body as set forth in claim 1, the substrate and the covering material are formed of an extensible raw material.
Also, a heat generating body as set forth in claim 9 is characterized in that in the heat generating body as set forth in claim 1, the heat generating composition molded body is sectioned by a sectioned part which is a heat seal part and is disposed in a plural number.
Also, a heat generating body as set forth in claim 10 is characterized in that in the heat generating body as set forth in claim 1, the exothermic substance in the heat generating composition comprising iron powder particles, and the iron powder particles have iron oxide film.
Also, a heat generating body as set forth in claim 11 is characterized in that in the the heat generating composition molded body is compression treated.
Also, a heat generating body as set forth in claim 12 is characterized in that in the heat generating body as set forth in claim 1, after heat sealing, at least a part of the heat generating composition molded body is moved into the temporary adhering part which is not heat sealed, thereby deadhering the temporary adhering part which is not heat sealed.
Also, a heat generating body as set forth in claim 13 is characterized in that in the heat generating body as set forth in claim 1, the heat generating body has a fixing measure on the exposed surface thereof.
As set forth in claim 14, a process for producing a heat generating body of the invention is characterized in that an air-permeable substrate and/or covering material at least has a heat sealable heat seal layer; a heat generating composition molded body resulting from molding of a heat generating composition containing, as essential components, an iron powder, a carbon component, a reaction accelerator and water and having a water mobility value of from 0.01 to 20 is laminated on the substrate; a hot melt based adhesive is provided as a sticky layer thereon by a melt blow system; the covering material is covered thereon; the heat generating composition molded body and/or the substrate is temporarily adhered to the covering material by temporary adhesion rolls; the periphery of the heat generating composition molded body is then heat sealed; and the seal part has a seal strength at 60°C of 0.8 kg/ 25 mm or more.

Also, a process for producing a heat generating body as set forth in claim 15 is characterized in that in the process for producing a heat generating body as set forth in claim 14, after temporarily adhering the outer circumstance of the heat generating body, the outer circumference of the heat generating body is heat sealed while retaining an air vent part.

Also, a process for producing a heat generating body as set forth in claim 16 is characterized in that in the process for producing a heat generating body as set forth in claim 14, heat sealing is achieved by using heat rolls having the heat seal part smaller than the temporary adhering part in the temporary adhesion rolls.

Also, a process for producing a heat generating body as set forth in claim 17 is characterized in that in the process for producing a heat generating body as set forth in claim 14, the heat generating composition molded body is moved into the temporary adhering part which is not heat sealed, thereby deadhering the temporary adhering part which is not heat sealed.

Also, it is preferable that in the heat generating body, the central part of the heat seal part is heat sealed, and the surroundings of the heat seal part are adhesive sealed.

Also, it is preferable that in the heat generating body, the sticky layer is laminated on the heat seal layer of the covering material.

Also, it is preferable that in the heat generating body, the sticky layer is laminated on the upper surface of the heat generating composition molded body.

Also, it is preferable that in the heat generating body, the melting point of a base polymer of the adhesive which constitutes the sticky layer is not higher than the melting point of a heat seal material which constitutes the heat seal layer.

Also, it is preferable that in the heat generating body, the fixing measure is an adhesive layer, and the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous substance, a moisturizer, a functional substance, and a mixture thereof.

Also, it is preferable that in the heat generating body, the thickness of the sticky layer is not more than the total thickness of the respective heat seal layers of the substrate and the covering material.

Also, it is preferable that in the heat generating body, the sticky layer has a void.

Also, it is preferable that in the heat generating body, the heat seal layer is constituted of an ethylene based heat seal material, and the sticky layer is constituted of a hot melt based adhesive layer.

Also, it is preferable that in the process for producing a heat generating body, the temporary adhesion roll is a die roll and/or a flat roll in which at least the surface thereof is flexible.

Also, it is preferable that in the process for producing a heat generating body, a pushing roll having a pushing part smaller than the heat generating composition molded body is used.

Also, it is preferable that in the process for producing a heat generating body, the heat generating composition molded body is moved along a collapsing plate.

[Advantages of the Invention]

The invention brings the following effects.

1. In the heat generating body according to the invention, upon temporary adhesion of a substrate and a covering material with a sticky layer and subsequent heat sealing, it is possible to surely achieve heat sealing without generating wrinkles and realize high-speed heat sealing.

2. In the case of sealing the substrate and the covering material in this way, not only it is only required to apply a pressure, but also heat fusion as in the conventional art is not needed. Accordingly, it is not necessary to think of any influence of thermal conduction of the substrate or covering material, the movement of a heat generating composition laminate can be easily prevented from occurring, and sealing can be surely achieved by next heat sealing. Thus, the seal failure is prevented so that the reliability of the heat generating body is remarkably improved.

3. In addition, since the heat generating body is constituted such that the substrate and the covering material are adhered with a sticky layer, the both can be temporarily adhered extremely easily only by applying a pressure, and next heat sealing can be easily achieved. As a result, higher line speed is realized, and the productivity is remarkably improved.

4. In addition, by employing, as the sticky layer, a layer which is compatible with a heat sealable layer, an adhesive and a heat seal material are well mixed without causing separation at the time of heat sealing, whereby it has become possible to achieve heat sealing more surely.

[Best Modes for Carrying Out the Invention]

The heat generating body of the invention is a heat generating body as formed by laminating a heat generating composition molded body resulting from molding of a heat generating composition containing, as essential components, an exothermic substance, a carbon component, a reaction accelerator and water and having a water mobility value of from 0.01 to 20 on a substrate, covering by a covering material and heat sealing the periphery of the heat generating composition molded body, wherein the substrate and the covering material are temporarily adhered with a sticky layer, the temporarily adhered portion is heat sealed with a heat seal layer which the substrate and/or the covering material has, the sticky layer component and the heat seal layer component are co-present in the heat seal part, and the heat seal part has a seal strength at 60°C of 0.8 kg/25 mm or more.

The invention designs to realize high speed of heat sealing and to seal stabilize the heat seal part, thereby bringing the following advantages.

1) Since the heat generating body is constituted such that the substrate and the covering material are adhered with a sticky layer, the both can be temporarily adhered extremely easily only by applying a pressure. As a result, the invention is very suitable for temporary adhesion for the purpose of realizing higher line speed.

2) Also, a heat seal device is not required so that simplification of production facilities can be realized. Moreover, in the case of carrying out heat fusion by heat sealing or heat bonding using a hot melt based bonding agent, while a heat source is necessary, since in adhesive sealing, the substrate and the covering material are sealed with an adhesive layer, a heat source such as electric power becomes unnecessary, thereby economizing on energy. As a result, it becomes possible to achieve temporary adhesion of the heat generating body or thermal sticking agent extremely economically.

3) When temporary adhesion of the surroundings of the substrate and the covering material is carried out with a sticky layer in this way, the heat generating composition molded body interposed between the substrate and the covering material does not cause deviation and can be moved to a next step at high speed.

4) In the case where the substrate and the covering material are heat sealed, since the movement of the heat generating composition molded body is controlled by temporary adhesion, it is possible to carry out heat sealing while following the movement of the surfaces of the heat seal rolls.

5) Since the adhesive which is used in the sticky layer is well compatible with the hot melt based bonding agent to be used for heat sealing, it is dispersed in the heat seal part so that it does not hinder heat sealing. Thus, an appropriate heat seal part can be formed.

6) Since the substrate and the covering material are brought into intimate contact with each other by temporary adhesion via the sticky layer, the heat seal part can be easily formed by heat sealing. Thus, it has become possible to achieve stable heat sealing at a high speed of 20 m/min or more, an aspect of which was difficult so far.

The "temporary adhesion" as referred to in the invention means weak pressure-sensitive bonding or adhesion for the purpose of holding the accommodated heat generating composition molded body until at least the substrate and the covering material are adhered to each other via a sticky layer and heat sealed.

Furthermore, the sticky layer is constituted of an adhesive and has a seal strength at 60°C of from 0.01 to 0.8 kg/25 mm. In this way, the movement of the heat generating composition molded body between the substrate and the covering material can be stopped, thereby making it possible to carry out high-speed heat sealing. In addition, if desired, the temporary adhesion may be carried out under warming. It is preferable that the warming is carried out by a treatment under pressure at a temperature of not higher than the melting point of a base polymer in a hot melt based adhesive which forms the sticky layer.

Though the temporarily adhered seal part is formed via the sticky layer, the adhesive which constitutes the sticky layer is not limited so far as it is a layer formed of a polymer composition which is tacky at the ordinary temperature and can achieve heat sealing after temporary adhesion.

Furthermore, the adhesive which constitutes the sticky layer to be used for temporary adhesion is preferably a non-hydrophilic adhesive. It is preferable that the adhesive which constitutes the sticky layer is well compatible with the heat seal material which constitutes the heat seal and that the melting point of a base polymer of the adhesive is not higher than the melting point of the heat seal material. In particular, the holt melt based bonding agent is preferably a hot melt based adhesive. Furthermore, in the case where the heat seal material is an olefin based raw material, an olefin based adhesive is enumerated as a preferred example of the adhesive.

Prior to the formation of the temporary adhering part of the invention, it is preferable that an adhesive is laminated on the substrate, the heat generating composition molded body, or the covering material. It is especially preferable that an adhesive which is well compatible with the heat seal layer is laminated on the periphery of at least the heat generating composition molded body on the substrate and/or the periphery of at least the heat generating composition molded body beneath the covering material.

That is, there are enumerated the case where a sticky layer is laminated on the periphery of the heat generating composition molded body on the substrate and/or the periphery of the heat generating composition molded body beneath the covering material; the case where a sticky layer is laminated on the entire surface on the substrate and/or the entire surface beneath the covering material; the case where a sticky layer is laminated on the entire surface on the substrate and/or the periphery of the heat generating compositionmoldedbodybeneath the covering material; the case where a sticky layer is laminated on the periphery of the heat generating composition molded body on the substrate and/or the entire surface beneath the covering material; and the case where such a sticky layer is partially laminated on the entire surfaces of the respective places. Furthermore, in the foregoing respective cases, an adhesive layer may be interspersed entirely or partially on the heat generating composition molded body.

In addition, in the case where a sticky layer is laminated on the periphery of the heat generating composition molded body on the substrate and/or the periphery of the heat generating composition molded body beneath the covering material, the sticky layer may be partially interspersed on the whole of a site from which the sticky layer is eliminated in the substrate and/or the covering material, namely over a corresponding region where the heat generating composition molded body is laminated.

The "seal strength at 60 °C" as referred to herein means an average value of respective maximum values obtained by subjecting three samples to a measurement by taking a specimen of 25 mm × 250 mm from a place of a subjective sealed sample to be measured for the seal strength, allowing the specimen to stand under circumstances at 60 °C for 5 minutes, grasping it under circumstances at 60 °C, and measuring a maximum strength at intervals of 10 mm and at a tensile speed of 300 mm/min.

The seal strength of the temporary adhering part is preferably 0.5 kg/25 mm or more, more preferably from 0.5 to 1 kg/ 25 mm, further preferably from 0.5 to 0.9 kg/25 mm, and still further preferably from 0.5 to 0.8 kg/25 mm under circumstances at 20 °C; and the seal strength at 60 °C is preferably less than 0.8 kg/25 mm, more preferably 0.01 kg/25 mm or more but less than 0.8 kg/25 mm, further preferably 0.01 kg/25 mm or more but less than 0. 5 kg/25 mm, and still further preferably 0.01 kg/25 mm or more but less than 0.4 kg/25 mm.

The sticky layer of the temporary adhering part is constituted of an adhesive, has a seal strength at 60 °C of from 0.01 to 0.8 kg/25 mm, is able to stop the movement of the heat generating composition molded body between the substrate and the covering material, and makes it possible to achieve high-speed heat seal. In addition, if desired, warming may be carried out at the time of temporary adhesion. It is preferable that the warming is carried out under pressure at a temperature of not higher than a melting point of a base polymer in a hot melt based adhesive for forming the adhesive layer.

The seal strength under circumstances at 20 °C of the heat seal part which has been heat sealed after the temporary adhesion is preferably 1.0 kg/25 mm or more, more preferably 1.2 kg/25 mm or more, further preferably 1.5 kg/25 mm or more, and still further preferably from 1.5 to 3 kg/25 mm. Furthermore, the seal strength at 60 °C under circumstances at 60 °C is preferably 0.8 kg/25 mm or more, more preferably 1.0 kg/25 mm or more, further preferably 1.2 kg/25 mm or more, and still further preferably 1.5 kg/25 mm or more. Here, the condition of the seal strength under circumstances at 20 °C is identical with that of the seal strength at 60 °C, except that the circumferential temperature for the measurement is 20 °C.

The range where the temporary adhering part is provided is not limited. The contact bonding surface may be any of partial contact bonding or entire contact bonding.

Furthermore, though its measure is not limited, too, the contact bonding can be preferably carried out by using a die plate or a planar or flat plate. Furthermore, the contact bonding can also be carried out by passing through die rolls or planar or flat rolls. The die rolls are constituted such that in order that only the temporary adhering part is contact bonded, the contact bonding part is formed in a contact bonding shape which is substantially similar to the periphery of the heat generating composition molded body; or that the contact bonding part is formed in such a manner that the a part of the surroundings of the heat generating composition molded body is contact bonded, whereas the heat generating composition molded body is not contact bonded. In other words, there is no limitation so far as the heat generating composition molded body is not contact bonded. Though the contact bonding surface may be a plain surface, taking into account slipperiness of a raw material or transportation, it is preferable that at least one surface of the contact bonding surface is an embossed surface. The shape of embossing is not particularly limited. Usually, examples thereof include a wavy form, a hexagonal form, a ring-like form, a polka-dotted form, and a reticulate form. A rate of the area of projections on the embossed surface is not particularly limited. Usually, it is preferably from 0.5 to 60 %. Furthermore, the contact bonding part may be provided partially or entirely against the temporary adhering part. The planar or flat rolls are one which is made of a flexible raw material and temporarily adheres at least the periphery of the heat generating composition molded body while applying a pressure to the whole of the heat generating body. The foregoing raw material is not limited so far as it can be deformed and temporarily adhered. Examples thereof include an expanded body, a felt, a woven fabric, a non-woven fabric, a rubber, and a balloon body.

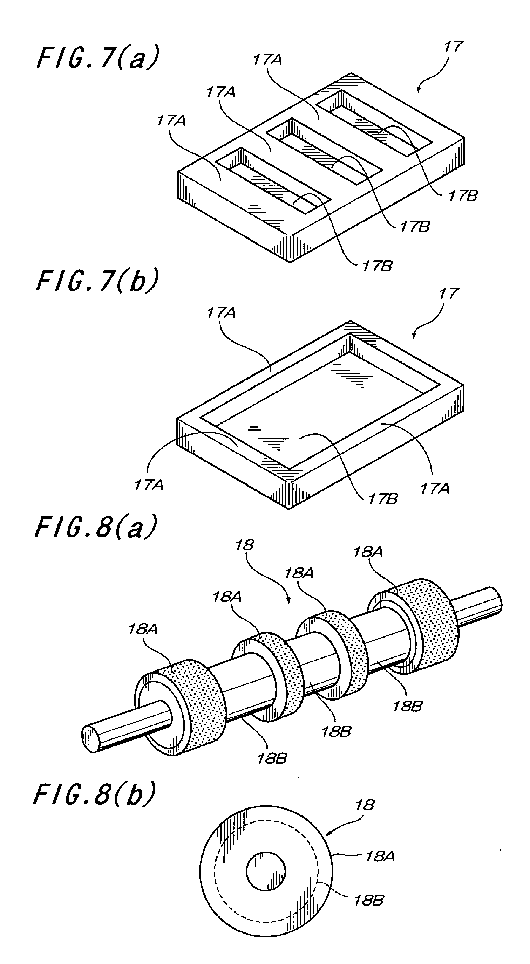

Furthermore, the temporary adhesion is carried out by using a temporary adhering tool, examples of which are shown in Figs. 7 to 13. However, the temporary adhering tool is not limited, but any tool can be used so far as it is able to achieve the temporary adhesion. In particular, in the case where the heat generating compositionmoldedbody is thick, it is preferred to temporarily adhere at least one member selected from a heat generating composition molded body and a substrate end part as shown in Fig. 8 depending upon the kind of a covering material to be used. The "substrate end part" as referred to herein means an end part of the substrate parallel to the MD direction of the heat generating composition molded body and a region which is parallel to the TD direction and in which the heat generating composition molded body is not present.

Furthermore, the temporary adhesion may be achieved by providing a sagging of the covering material between at least one heat generating composition molded body and at least one adjacent heat generating composition molded body. The sagging at the time of temporary adhesion may be provided in one direction or plural directions. This is because cutting in seal can be prevented by a gentle sagging.

Furthermore, it is possible to embody a thin-width heat seal part by combining temporary adhesion, heat sealing and the movement of a heat generating composition at a higher speed. In the case where an exothermic part is constituted of a sectional exothermic part and a sectioned part which is the seal part, when the formation of the sectioned part is achieved only by heat sealing, it was required to make the sectioned part large positioned between a sectional exothermic part and a sectional exothermic part due to a critical width of the heat seal. However, when the space between a sectional exothermic part and a sectional exothermic part becomes large, a heat insulation effect between the sectional exothermic parts is reduced so that the exothermic time becomes short. Accordingly, this method for softening the heat generating body involved a problem in view of the exothermic duration. By employing a combination of temporary adhesion, heat sealing and the movement of the heat generating composition according to the invention, when heat sealing is carried out after the temporary adhesion, it becomes possible to form a thin-width heat seal part at high speed without scattering the heat generating composition into the heat seal part; and after forming the heat seal part, by using a pushing roll, etc., the heat generating composition is moved into a region which is the temporary adhering part but is not heat sealed, the non-heat seal part is deadhered, thereby making it possible to make the width of the sectioned part small. In this way, it has become possible to embody a flexible heat generating body without shortening the exothermic duration of the heat generating body.

In the invention, as a heat seal material constituting a heat seal layer, a single rawmaterial may be used, oracomposite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins,such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.

The thickness of the heat seal layer is not limited so far as heat sealing can be achieved. It is usually from 5 to 1,000 $\mu$m, preferably from 10 to 500 $\mu$m$^2$, and more preferably from 15 to 250 $\mu$m. When the thickness of the heat seal layer is less than 5 $\mu$m, there is some possibility that a prescribed adhesive strength is not obtained. On the other hand, when it exceeds 1,000 $\mu$m, the heat seal rate becomes slow.

The foregoing adhesive is not limited so far as it is a layer formed of a polymer composition which is tacky at the ordinary temperature, it is well compatible with the heat seal layer and it can achieve heat sealing after the temporary adhesion. Specific examples thereof include a solvent type adhesive and a hot melt based adhesive.

Furthermore, since in the expanded adhesive layer, a gas in the pore causes elastic deformation, elasticity, stretchability and flexibility are remarkably enhanced. As a result, not only flexibility of a packaging material made of a substrate and a covering material, especially flexibility of a seal part between the substrate and the covering material is remarkably enhanced, but also a feeling for use is much more enhanced.

Examples of a method for expanding a hot melt based adhesive include a chemical expansion method and a physical expansion method. The chemical expansion method is a method in which an inorganic expanding agent or an organic expanding agent or a mixture thereof is used and expansion is carried out by utilizing a nitrogen gas or a carbonic acid gas as generated during its decomposition reaction; and the "physical expansion" as referred to herein is a method in which a gas such as compressed air, compressed nitrogen and a compressed carbonic acid gas is incorporated into the adhesive by a physical force.

Of these expansion methods, physical expansion methods as described in JP-B-60-3350, JP-A-62-87267, JP-B-63-17295, and JP-A-1-59023 are especially recommended for the reasons as described below.

That is, according to the physical expansion method, by bringing a pressurized gas into contact with a hot melt based adhesive to be injected in the air from a nozzle, the adhesive is blown away in a finely fibrous state, thereby forming fine continuous pores or closed cells.

Since its expansion ratio depends upon a temperature related to the viscosity of the adhesive and a pressure of the pressurized gas, it is easy to set up the temperature condition and pressure condition for the purpose of obtaining an adhesive layer of a prescribed expansion ratio as compared with the case of using an expanding agent.

Furthermore, in this physical expansion method, for the purpose of stabilizing the expansion ratio, it is only required to stabilize an injection amount or injection rate of the adhesive from a nozzle and a pressure of the pressurized air. Thus, it is easy to obtain a uniform adhesive layer.

In the case of carrying out this physical expansion method, in order to increase a control precision of the expansion ratio, it is preferred to bring the compressed gas into contact with the adhesive as injected from the nozzle as fast as possible. Accordingly, for example, it is preferred to provide a gas port for injecting the compressed gas in the surrounding of an injection port from which the adhesive is injected, thereby bringing the compressed gas into contact with the adhesive at the same time of injecting the adhesive.

Furthermore, for the purpose of shortening the painting time by simultaneously injecting the adhesive into a wide range as far as possible, it is preferable that the injection port from which the adhesive is injected is formed in a slit form or a number of injection ports of the adhesive are provided adjacent to each other in series.

Furthermore, in the invention, it is beneficial that the adhesive is formed of a hot melt type polymer substance, an alicyclic petroleum resin and a softening agent, and this adhesive is expanded by the foregoing methods.

In the invention, as the hot melt type polymer substance, those as described previously are enumerated. This hot melt type polymer substance is a base polymer in the adhesive, and an expanded body of the adhesive as formed by using this has excellent shape holding properties, has initial tackiness, is good in adhesiveness at the time of the normal temperature or at the time of warming, and is stable with respect to adhesive strength after the adhesion.

Furthermore, in the invention, the alicyclic petroleum resin is a tackifier, and its combination with the hot melt type polymer substance gives rise to prescribed adhesive characteristics.

The "alicyclic petroleum resin" as referred to herein is a petroleum resin having a cyclic skeleton. Specific examples thereof include rosins, dehydrated rosins, glycerin esters of a dehydrated rosin, glycerin esters of a gum rosin, hydrogenated rosins, methyl esters of a hydrogenated rosin, glycerin esters of a hydrogenated rosin, pentaerythritol rosins of a hydrogenated rosin, polymerized rosins, glycerin esters of a polymerized rosin, chroman-indene resins, hydrogenated petroleum resins, maleic anhydride modified rosins, rosin derivatives, and C-5 based petroleum resins. For the purpose of imparting a prescribed adhesive strength to the sticking agent, the petroleum resin is properly used singly or in combination of two or more kinds thereof.

The substrate of the invention is substantially planar and does not have an accommodating pocket; the covering material covers the heat generating composition provided on the substrate; the sectional exothermic part which is constituted by heat sealing the periphery of the heat generating composition is made of two or more plural sectional exothermic parts; the respective sectional exothermic parts are disposed at intervals by the sectioned part which is a heat seal part; and the exothermic part is formed of a gathering of the foregoing sectional exothermic parts. Here, in the invention, the substrate and the covering material are not distinguished from each other depending upon a raw material constitution; but a raw material on which the heat generating composition molded body is laminated is defined as a substrate, and a raw material which is then covered on the substrate or heat generating composition molded body is defined as a covering material.

The term "substantially planar" as referred to in the invention means a planar surface not having an accommodating concave such as an accommodating pocket, an accommodating section, and an accommodating zone as provided in advance for the purpose of accommodating the heat generating composition. Accordingly, irregularities which do not intentionally accommodate the heat generating composition may be present.

The "pocket" as referred to in the invention is an accommodating pocket which is provided in advance for the purpose of accommodating the heat generating composition and is a pocket as described in JP-T-2001-507593. Since irregularities which are not used for intentionally accommodating the heat generating composition molded body are not the pocket, even when such irregularities are present on a substrate, it is to be noted that such a substrate is defined as a substantially planar substrate.

The "accommodating section" as referred to herein is an accommodating section for accommodation as provided in advance on the packaging material for the purpose of accommodating the heat generating composition and is an accommodating section as described in Japanese Patent No. 3,161, 605 and JP-T-11-508314. Since irregularities which are not used for intentionally accommodating the heat generating composition molded body are not the accommodating section, even when such irregularities are present on a substrate, it is to be noted that such a substrate is defined as a substantially planar substrate.

The "accommodating zone" as referred to herein is an accommodating zone for accommodation as provided in advance on the packaging material for the purpose of accommodating the heat generating composition and is an accommodating zone as described in Japanese Patent No. 3, 161, 605 and JP-T-11-508314. Since irregularities which are not used for intentionally accommodating the heat generating composition molded body are not the accommodating zone, even when such irregularities are present on a substrate, it is to be noted that such a substrate is defined as a substantially planar substrate.

The raw material which constitutes the substrate, the covering material or the underlay material is not limited so far as it functions as an accommodating bag of the heat generating composition. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, expanded raw materials, non-expanded raw materials, non-heat sealable rawmaterials, and heat sealable raw materials. The raw material can be properly used depending upon a desired utility in a desired form such as films, sheets, non-woven fabrics, woven fabrics, and composites thereof.

Usually, the substrate is made of an air-impermeable film or sheet, and the covering material is made of an air-permeable film or sheet or non-woven fabric, and vice versa. The both may be air-permeable. Furthermore, in the underlay material, air permeability or air impermeability may be properly used for different purposes.

The substrate or covering material may be of a single-layered structure or multilayered structure, and its structure is not limited. As examples of the multilayered structure, the substrate is made of layer A/layer B, layer A/layer B/layer C, or layer A/layer B/layer C/layer D; and the covering material is made of layer F/layer G, layer E/layer F/layer G, or layer F/layer H/layer G.

Incidentally, examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), and wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons) ; and examples of the layer H include non-water absorptive papers and water absorptive papers. Examples of the substrate or covering material include heat seal layer made of polyethylene obtained by using a metallocene catalyst/polyethylene film, polyethylene-made heat seal layer/polypropylene film, heat seal layer made of polyethylene obtained by using a metallocene catalyst/polyethylene film/adhesive layer/separator, EVA-made heat seal layer/polypropylene film/adhesive layer/separator, polyethylene-made heat seal layer/polyethylene film/nylon non-woven fabric, EVA-made heat seal layer/polyethylene film/nylon non-woven fabric, polyethylene-made heat seal layer/polypropylene film/polypropylene non-woven fabric, non-woven fabric/porous film, non-woven fabric/ paper, perforated (provided by a needle or laser) film/porous film, non-woven fabric/paper, porous film/perforated (provided by a needle or laser) film, non-woven fabric/paper, and porous film/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. The adhesive layer may be provided on such a packaging material.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system, and a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the coveringmaterial, the underlay material, and the raw material constituting the same largely varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a stretched polyolefin film (for example, a biaxially stretched polypropylene film).

The air-permeable raw material is not limited so far as it is air-permeable. Examples thereof include air-permeable films (for example, porous films and perforated films); materials having air permeability by themselves (for example, papers and non-woven fabrics) ; materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat contact bonding; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10, 000 $g/m^2/4$ hr, preferably from 70 to 5, 000 $g/m^2/24$ hr, more preferably from 100 to 2,000 $g/m^2/24$ hr, and further preferably from 100 to 700 $g/m^2/24$ hr in terms of moisture permeability by the Lyssy method.

When the moisture permeability is less than 50, the heat value is small and a sufficient thermal effect is not obtained, and therefore, such is not preferable. On the other hand, when it exceeds 10,000 $g/m^2/24$ hr, the exothermic temperature is high so that a problem in safety may possibly be generated, and therefore, such is not preferable. However, there is no limitation even when the moisture permeability exceeds 10,000 $g/m^2/24$ hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials thereof with or blended materials of such a stretchable raw material and a non-stretchable raw material or fabrics constituted of a combination of these materials, films, and yarns.

The porous film can be properly selected among porous films obtained by stretching a film made of a polyolefin based resin (for example, polyethylene, linear low density polyethylene, and polypropylene) or a fluorine based resin (for example, polytetrafluoroethylene) and a filler.

As the non-woven fabric, single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful.

Furthermore, in the components to be used in the moldable heat generating composition and the heat generating body of the invention and the packaging materials such as the substrate, the covering material and the underlay material, biodegradable raw materials can be used in addition to conventionally employed raw materials.

As the heat generating composition, it is preferred to use a heat generating composition capable of causing an exothermic reaction upon contact with air, which contains, as essential components, an exothermic substance such as iron, a carbon component, a reaction accelerator and water, contains surplus water so as to have a water mobility value of from 0.01 to 20 and has moldability due to the surplus water and in which the water in the heat generating composition does not function as a barrier layer.

Furthermore, it is preferable that the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

As the "iron powder" as referred to herein, usual iron powders, iron alloy powders and active iron powders such as iron powders comprising particles, a surface of each of which is at least partially covered with an oxygen-containing film , and iron alloy powders comprising particles , a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed. The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;
(B) an active iron powder in which the content of wustite is from 2 to 50 % by weight in terms of an X-ray peak intensity ratio to iron;
(C) an iron powder having an iron oxide film having a thickness of 3 nm or more on the surface thereof; and
(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 $\mu$m, more preferably from 30 nm to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 $\mu$m or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0.5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.

Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.

Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;

(2) a process for producing a heat generating mixture by subjecting a reaction mixture of an iron powder, a reaction accelerator, an acidic substance and water in an oxidizing gas atmosphere to a self-exothermic reaction;

(3) a process for producing a heat generating mixture by subjecting a reaction mixture of an iron powder, a reaction accelerator, a carbon component and water in an oxidizing gas atmosphere to a self-exothermic reaction;

(4) a process for producing a heat generating mixture by subjecting a reaction mixture of an iron powder, a reaction accelerator, an acidic substance, a carbon component and water in an oxidizing gas atmosphere to a self-exothermic reaction;

(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;

(6) a process for producing a heat generating mixture by carrying out the method as set forth above in any one of (1) to (5) under circumstances heated so as to have temperature of at least 10 °C higher than the circumferential temperature;

(7) a process for producing a heat generating mixture by carrying out the method as set forth above in any one of (1) to (6) by blowing an oxidizing gas;

(8) a process for producing a heat generating mixture by carrying out the method as set forth above in (7) by blowing the oxidizing gas heated so as to have a temperature of at least 10 °C higher than the circumferential temperature;

(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;

(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;

(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and

(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.

Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.

Furthermore, the contact treatment with an oxidizing gas may be carried out with or without stirring in a fluidized or non-fluidized state and may be carried out in a batch or continuous system.

Examples of the final heat generating composition include:

1) a heat generating composition containing, as a heat generating composition raw material, a heat generating mixture produced in the process as set forth above in any one of (1) to (12);

2) a heat generating composition obtained by adding other components to the heat generating composition as set forth above in 1); and

3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1, 000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.

2) A magnet is provided in the vicinity of a central part of the back side of a polyvinyl chloride-made supporting plate (3 mm in thickness × 600 mm in length × 600 mm in width) of a footed supporting table so as to cover a cavity shape of a molding die.

3) A temperature sensor is placed on the central part of the supporting plate.

4) A polyethylene film (25 μm in thickness × 250 mm in length × 200 mm in width) as provided with an adhesive layer having a thickness of about 80 μm is stuck onto the supporting plate via a sticky layer such that the center of the polyethylene film is positioned at the sensor.

5) The heat generating composition is taken out from the outer bag.

6) A template (250 mm in length × 200 mm in width) having a cavity (80 mm in length × 50 mm in width × 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.

In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.

The thickness of the oxygen-containing film of a prescribed amount or the oxygen-containing film of iron powder containing wustite and the amount of wustite are applied to the heat generating composition or the heat generating composition molded body at the time of lamination.

An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.

Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50, in particular from 0.01 to 20. The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.

2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.

3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

In addition, if desired, at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be further added to the heat generating composition.

Furthermore, in the heat generating composition of the invention or the like, although there is no particular limitation for the blending ratio thereof, it is preferred to select the blending ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the amount of the water absorptive polymer is from 0.01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in blending ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0.01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight, respectively. Incidentally, a magnetic body may further be blended, and its blending ratio may be properly determined depending upon the desire.

Incidentally, these blending ratios can also be applied in a reaction mixture and a heat generating mixture. Furthermore, a water mobility value of the reaction mixture is usually less than 0.01.

As the water, one from a proper source may be employed. Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 3 to 20 % by weight, further preferably from 4 to 15 % by weight, and still further preferably from 7 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as woodmeal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth)acrylate and a (meth) acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly (meth) acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, NaOH, KOH, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

Examples of the oxidizing agent include nitrates, oxides, peroxides, halogenated oxygen acid salts, permanganates, and chromates.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenolethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

The process for producing a heat generating body according to the invention is a process for producing a heat generating body which is characterized by laminating a heat generating compositionmoldedbody resulting frommolding a heat generating composition containing, as essential components, an iron powder, a carbon component, a reaction accelerator and water on a substrate, providing thereon an adhesive as an air-permeable sticky layer by a melt blow system or the like, and further covering a covering material on the substrate and the heat generating composition molded body; or covering a covering material provided with an adhesive as an air-permeable sticky layer by a melt blow system or the like, carrying out contact bonding by temporary adhesion rolls, thereby temporarily adhering the substrate and the heat generating composition molded body to the covering material, and heat sealing the substrate in the periphery of the heat generating composition molded body and the covering material by heat seal rolls, thereby forming a heat seal part. Moreover, at least one of the substrate and the covering material is air-permeable. Incidentally, an air-permeable sticky layer may be provided in the heat seal part side of the covering material. An air-permeable sticky layer may be provided for the heat generating composition molded body as provided on the substrate by a melt blow method or the like. Furthermore, the temporary adhesion roll may be at least one member selected from a die roll and a flat roll in which at least the surface thereof is flexible. The surface of the roll may be any of a plain surface, a patterned surface or a mixture of plain and patterned surfaces.

Next, the temporary adhesion will be described with reference to Figs. 7 to 13.

Fig. 7(a) and Fig. 7(b) each shows an example of a temporary adhesion plate. A heat generating composition molded body or the like is accommodated in a concave 17B, and a temporary adhering part is formed by 17A.

Fig. 8(a) is an oblique view of a temporary adhesion roll 18 in the MD direction; and Fig. 8(b) is a side view thereof. In the drawings, a temporary adhering part is formed by 18A, and a heat generating composition molded body or the like is accommodated in 18B.

Fig. 9(a) is an oblique view of a roll 18 for temporarily adhering an end part of a substrate in the MD direction or an end part of a substrate in the MD direction and a top of a heat generating composition molded body to a covering material.

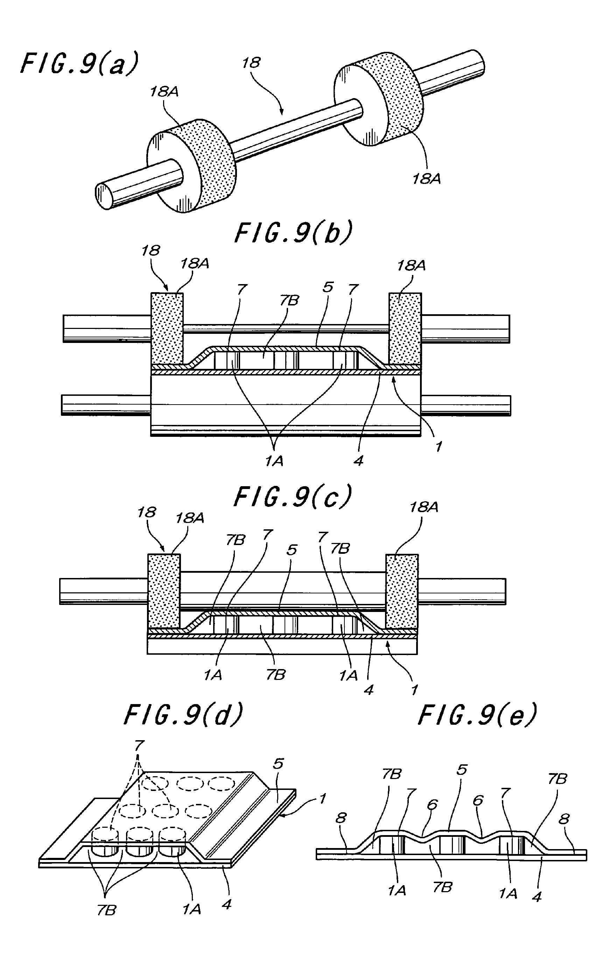

Fig. 9(b) is a cross-sectional view showing a roll 18 for temporarily adhering an end part of a substrate 4 in the MD direction to a covering material 5 and a part of a heat generating body 1. Fig. 9(c) is a cross-sectional view showing a roll 18 for temporarily adhering an end part of a substrate in the MD direction and a top of a heat generating composition molded body 1A to a covering material 5 and a part of a heat generating body 1. Fig. 9(d) is an oblique view showing a part of a heat generating body 1 in which an end part of a substrate in the MD direction and a top of a heat generating composition molded body 7 are adhered to a covering material 5. In Fig. 9(a), a temporary adhering part is formed by 18A. Incidentally, the heat generating composition molded body or the like is accommodated in a space between adjacent 18A and 18A; and in Fig. 9(b), the heat generating composition molded body is not pressed; in Fig. 9(c), the heat generating composition molded body is pressed. In Fig. 9(d), the end part of the substrate in the MD direction is in a temporarily adhered state by the temporary adhesion roll 18, and thereafter, the surroundings of the heat generating body including a space 7B between the adjacent heat generating composition molded bodies 7 and the foregoing temporary adhering part are heat sealed by a heat seal roll. In Fig. 9(e), a covering material is sagged between the heat generating composition molded bodies 7 and 7 by a temporary adhesion roll, the surroundings of the heat generating body are in a temporarily adhered state, and thereafter, the surroundings of the heat generating body including a space 7B between the adjacent heat generating composition molded bodies 7 and 7 and the foregoing temporary adhering part are heat sealed. It is possible to prevent cutting in seal by this sagging.

Fig. 10 is an oblique view of a temporary adhesion roll 18 in the TD direction, and a temporary adhering part is formed by 18A.

Fig. 11 is a plan view of a temporary adhesion roll 18 having a rectangular temporary adhering part, and the temporary adhering part is formed by 18A. When pressed by this roll 18, as shown in Fig. 12, a temporary adhering part 7 is formed and then heat sealed together with a portion 7A which is not temporarily adhered.

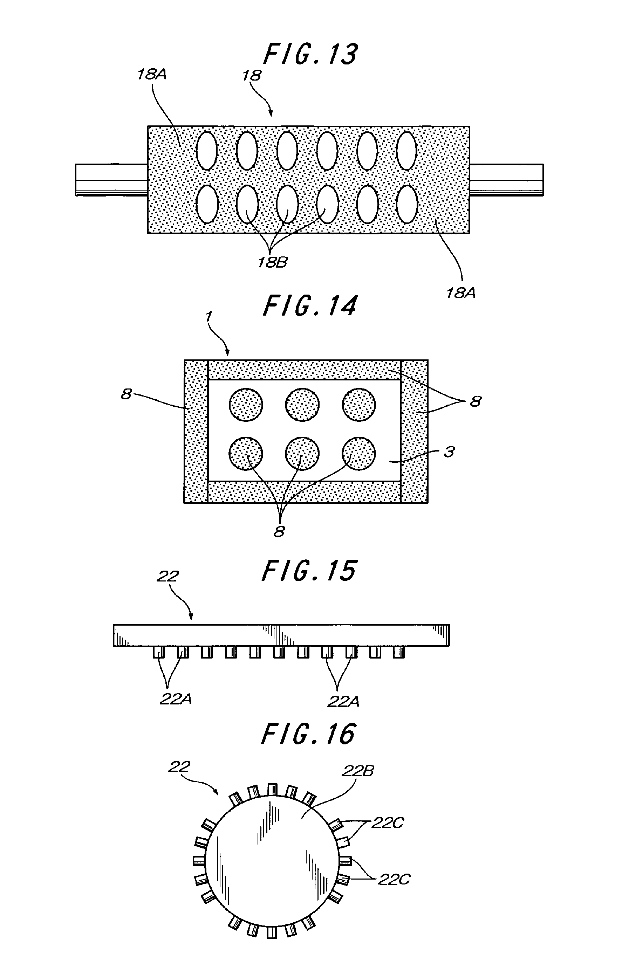

Further, Fig. 13 is a plan view of a temporary adhesion roll 18 having an elliptical exothermic part.

Fig. 14 is a plan view of a heat generating body having a circular sectioned part in an exothermic part as temporarily adhered by a temporary adhesion roll in which the elliptical portion of the temporary adhesion roll as shown in Fig. 13 is formed in a true circle shape, which is provided with a heat seal 8 after the temporary adhesion.

Figs. 15 to 17 are each a view explaining collapse and/or deadhesion of a heat generating body.

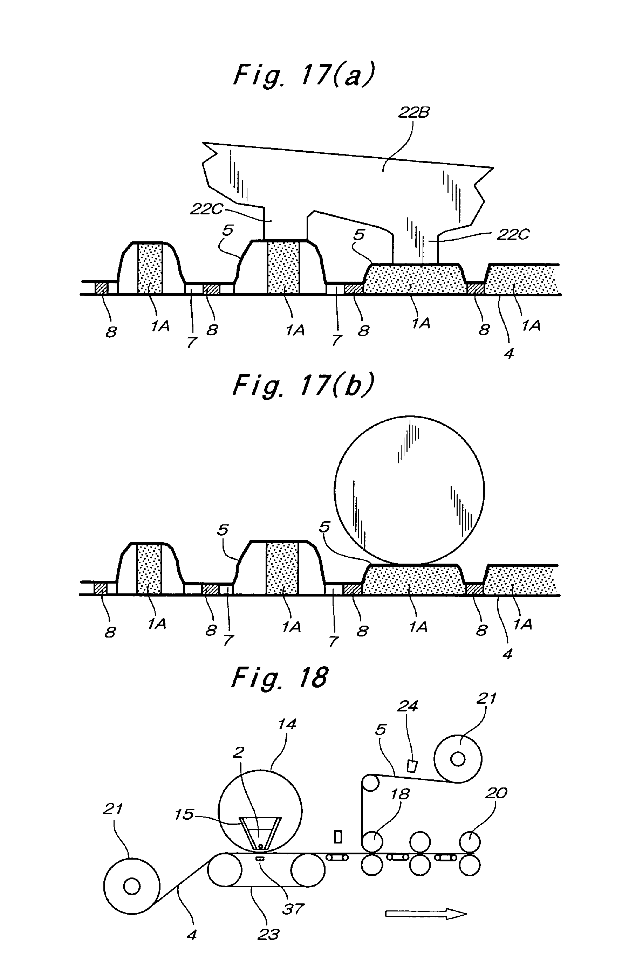

Fig. 15 is a cross-sectional view of a deadhesion plate 22 having a deadhering convex 22A and capable of achieving collapse and/or deadhesion. As shown in Fig. 17, a heat generating composition molded body or the like is pressed by this deadhering convex 22A via a covering material 5. Also, Fig. 16 is a cross-sectional view of a deadhesion plate 22 provided with a deadhering convex 22C as in Fig. 15 on the surface of a circular roll and capable of achieving collapse and/or deadhesion.

Fig. 17(a) is a schematic cross-sectional view showing collapse and deadhesion using a deadhesion roll 22B having a deadhering convex 22C and capable of achieving collapse and/or deadhesion; and as shown in the left-hand portion of this drawing, a heat generating composition molded body 1A is covered by a covering material 5, and a space is formed in the periphery thereof. Furthermore, a heat seal 8 is partially provided in the covering material 5 and a substrate 4. In this state, when the deadhering convex 22C presses the upper surface of the covering material 5, the heat generating composition molded body is collapsed and accommodated within the foregoing space; and as shown in the right-hand portion of this drawing, the heat generating composition molded body 3 is pushed into the heat seal part 8. Incidentally, Fig. 17(b) shows an example not having the deadhering convex 22C.

After these seal steps, a heat generating body is produced through a cutting step, etc. These seal step and cutting step and the like may be properly selected and employed from conventional methods and devices. Incidentally, in the case of a molding system, there are enumerated force-through molding using a trimming die, trimming die cast molding, and cast molding using a concave casting die.

According to this method, since the periphery of the substrate and the covering material are temporarily adhered with an adhesive layer and then heat sealed, the heat generating composition molded body does not cause deviation so that complicated heat sealing can be achieved at high speed.

With respect to the production process of a heat generating body according to the molding system, there are a conventional heat generating body in which the exothermic part is made of a single part and a heat generating body having a gathered exothermic part in which the exothermic part is sectioned and made of two or more sectional parts.

As the production process of such a heat generating body, any molding method using a die is employable. Examples thereof include a force-through molding method and a cast molding method. In the case where the exothermic part is made of a single part, molding may be carried out by using a trimming die or a concave casting die capable of molding a single exothermic part. In the case where the exothermic part is sectioned and made of two or more sectional parts, molding may be carried out by using a trimming die or a concave casting die capable of molding a gathered exothermic part made of two or more plural sectional exothermic parts. In the production of a heat generating body according to the molding system, it is possible to produce a heat generating body at a speed of three times or more as compared with a filling system. Thus, by employing the molding system, it is possible to largely reduce costs due to an improvement of the production speed. However, a material cannot be used as the heat generating composition to be used unless it is a moldable heat generating composition.

The "force-through molding method" as referred to herein means, for example, a continuous formation method in which by using a molding machine for laminating a heat generating composition molded body having a trimming die shape on a longitudinal substrate by using a trimming die and a rotary seal unit capable of covering the laminate by a longitudinal covering material and sealing (by heat sealing, contact bonding sealing, or heat contact boding sealing) a desired sectioned part and the peripheries of the substrate and the covering material, the peripheries of the heat generating composition molded body and a necessary part of the sectioned part are heat sealed via the seal unit and subjected to a seal treatment.

The "trimming die cast molding" as referred to herein means a method in which by using a trimming die, one surface of a cavity is covered by a roll, etc., a heat generating composition is filled in the cavity, a longitudinal substrate is then placed on the other surface, and a heat generating composition molded body is laminated on the substrate.

The "cast molding method" as referred to herein means, for example, a continuous molding method in which by using a molding machine for laminating a heat generating composition molded body on a longitudinal substrate by filling in a concave and moving into a substrate by a drum-type body of rotation having a concave and a rotary seal unit capable of covering the laminate by a longitudinal covering material and sealing (by heat sealing, contact bond sealing, or heat contact bond sealing) a desired sectioned part and the peripheries of the substrate and the covering material, the peripheries of the active heat generating composition molded body and a necessary part of the sectioned part are heat sealed via the seal unit and subjected to a seal treatment.

Furthermore, examples of the heat generating body using the heat generating composition include a heat generating body which is constituted such that the heat generating composition moldedbody is laminated between a substrate and an air-permeable packaging material constituted of a covering material.

The temporary adhesion can be carried out by using a temporary adhesion plate, a temporary adhesion roll, etc., covering it by the substrate and/or the heat generating composition and/or the heat generating composition molded body and a covering agent, and contact bonding a desired region by pressurization, pressurization under heating, etc. The pressurization under heating can be carried out by passing through a heat press machine or heat rolls. The pressurization or pressurization under heating can be carried out by using a plain or flat roll. However, in order to enhance a shape fixing effect while keeping the flexibility of a sheet-like material, it is preferable that at least one surface of the pressurizing surface or pressurizing surface under heating is embossed. The shape of embossing is not particularly limited. Usually, examples thereof include a wavy form, a hexagonal form, a ring-like form, a polka-dotted form, and a reticulate form. It is preferable that the heat generating composition powder easily keeps away into a non-compressed part at the time of pressurization or pressurization under heating. A rate of the area of projections on the embossed surface is not particularly limited. It is usually from 0.5 to 60.0 %, and preferably from 5.0 to 40.0 %. Furthermore, with respect to the material quality of the surface of the temporary plate and temporary roll, materials ranging from a rigid one to a flexible one can be used. In the case of the planar or flat roll, ones in which at least the surface thereof is flexible and the surface can be deformed are preferable. Examples thereof include raw materials such as a felt, a non-woven fabric, and a flexible rubber.

The temperature and pressure condition for the pressurization or pressurization under heating varies depending upon the material quality of the substrate or covering material and the softening temperature and/or the melting point of the adhesive or heat seal material. For example, in the case of using a heat roll, usually, the temperature is from about 70 to 300°C, and the linear pressure is from about 0.1 to 250 kg/cm. In this way, the heat seal material on the surface of the laminate which comes into contact with a projected part in the pressurized state is molten and fixed with respect to the shape.

Furthermore, the thickness of the sticky layer is not limited so far as the temporary adhesion and the heat sealing can be achieved. It is preferable that the total thickness of the heat seal layers of the substrate and the covering material is the thickness of the adhesive layer or more.

The "deadhesion" as referred to herein means that the state that the substrate and the covering material are adhered to each other via the sticky layer is removed, thereby releasing the adhesive state between the substrate and the covering material. It does not matter whether or not after deadhesion, an inclusion is present between the substrate and the covering material. There is enumerated a method in which a part of the temporary adhering part where the substrate and the covering material are temporarily adhered to each other via the sticky layer is heat sealed and the heat generating composition is moved into a non-heat sealed region between the substrate and the covering material, thereby making the space between the substrate and the covering material in a non-adhesive state.

What compatibility at the time of heat sealing is good means that when heat sealed, the heat sealing is not hindered and separation does not occur. That is, in the heat seal part, the sticky layer and the heat seal layer are not necessarily required to be completely fused, but it is only required that the heat seal strength after heat sealing is higher than the contact bonding seal strength. It is to be noted that if air does not invade into the heat generating composition from the heat seal part, the substrate and the covering material are heat sealed.

Furthermore, after temporary adhesion, by heat sealing in a heat seal width narrower than the temporary adhesion width and then deadhering the non-heat sealed temporary adhering part, heat sealing is completed in a substantially narrow heat seal width. According to the invention, it has become possible to achieve heat sealing in an extra-fine width. In particular, in a heat generating body having an exothermic part constituted of a sectional exothermic part and a sectioned part (heat seal part), such is an important structure and technology in the case of making flexibility consistent with exothermic characteristics. When the sectioned part width which is the heat seal width is large, the space between the sectional exothermic parts is widened, whereby unevenness in temperature of the exothermic part becomes large.

According to the invention, it becomes possible to prepare a heat generating body having an exothermic part wherein the space between the sectional exothermic parts is made minimum, and the sectional exothermic parts are not separated from so far. Thus, a heat insulation effect between the sectional exothermic parts is increased so that the exothermic time becomes long.

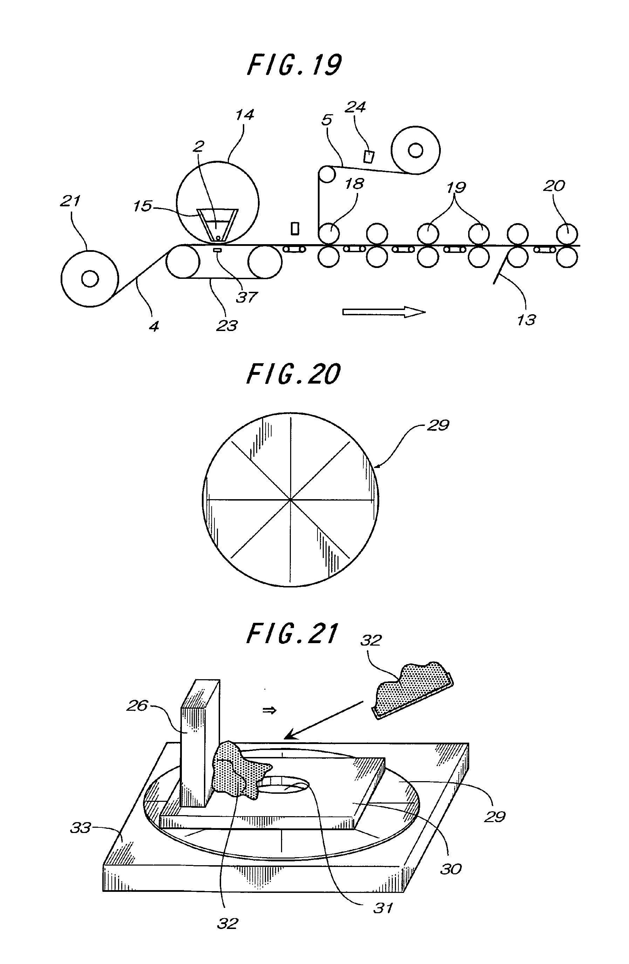

The "water mobility value" as referred to herein is a value showing an amount of surplus water which can transfer to the outside of the heat generating composition in water present in the heat generating composition. This water mobility value will be described below with reference to Figs. 20 to 24.

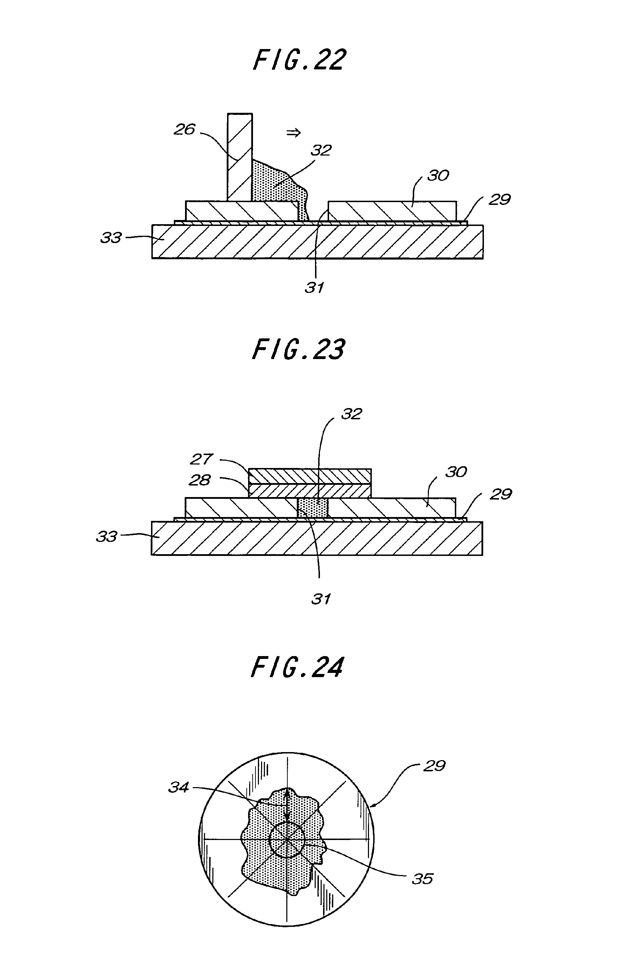

As shown in Fig. 20, a filter paper 29 of No. 2 (second class of JIS P3801) in which eight lines are drawn radiating from the central point with an interval of 45° is placed on a stainless steel plate 33 as shown in Figs. 21 and 22; a template 30 having a size of 150 mm in length × 100 mm in width and having a hollow cylindrical hole 31 having a size of 20 mm in inner diameter × 8 mm in height is placed in the center of the filter paper 29; a sample 32 is placed in the vicinity of the hollow cylindrical hole 31; and a stuffer plate 26 is moved on and along the template 30 and inserted into the hollow cylindrical hole 31 while stuffing the sample 32, thereby leveling the sample (force-in die molding).

Next, as shown in Fig. 23, a non-water absorptive 70 μm-thick polyethylene film 28 is placed so as to cover the hole 31, and a flat plate 27 made of stainless steel having a size of 5 mm in thickness × 150 mm in length × 150 mm in width is further placed thereon and held for 5 minutes such that an exothermic reaction is not caused.

Thereafter, a shown in Fig. 23, the filter paper 29 is taken out, and an oozed-out locus of the water or aqueous solution is read as a distance 34 (unit: mm) from a periphery 35 as an edge of the hollow cylindrical hole to an oozed-out tip along the radiating lines. Similarly, a distance 34 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter × 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

```
(Water mobility value) = {[Water content value (mm)]/

[(Real water content value (mm))] × 100
```

With respect to the same sample, five points are measured, and the five water mobility values are averaged, thereby defining an average value thereof as a water mobility value of the sample.

In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 $\mu$m, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 $\mu$m, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness × 200 mm in length × 200 mm in width and having a cavity as treated by R5 in four corners of 60 mm in length × 40 mm in width in a central part thereof) and a fixable leveling plate are disposed above a travelable endless belt, and magnets (two magnets having a size of 12.5 mm in thickness × 24 mm in length × 24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness $\times$ 200 mm in length $\times$ 200 mm in width is placed on the endless belt of the measurement device, a polyethylene film having a size of 70 $\mu$m in thickness $\times$ 200 mm in length $\times$ 200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1. 8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (5 mm in thickness $\times$ 600 mm in length $\times$ 600 mm in width) of a footed supporting table so as to cover a cavity shape of a molding die.

2) A temperature sensor is placed on the central part the surface of the supporting plate.

3) A polyethylene film (25 $\mu$m in thickness $\times$ 250 mm in length $\times$ 200 mm in width) as provided with an adhesive layer having a thickness of about 80 $\mu$m is stuck onto the supporting plate via a sticky layer such that the center of the polyethylene film is positioned at the sensor.

4) On an underlay plate (280 mm in length $\times$ 150 mm in width $\times$ 50 $\mu$m to 2 mm in thickness), a polyethylene film (230 mm in length $\times$ 155 mm in width $\times$ 25 $\mu$m to 100 $\mu$m in thickness) is placed such that one end of the polyethylene film is projected by about 20 mm outside the underlay plate and that one end thereof in the length direction is substantially coincident with one end of the underlay plate.

5) A template (230 mm in length $\times$ 120 mm in width $\times$ 3 mm in thickness) having a cavity (80 mm in length $\times$ 50 mm in width $\times$ 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.
8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.
9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.
The thickness after compression is preferably from 50 to 99.5 %, more preferably from 60 to 99.5 %, and further preferably from 60 to 95 % of the die thickness.
Incidentally, in the invention, it is to be noted that the heat generating composition molded body includes a heat generating composition compressed body.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.
In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.
Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

The "heat generating mixture" as referred to herein is a material obtained by subjecting a reaction mixture containing, as essential components, an iron powder, a carbon component, a reaction accelerator and water and having a water content of from 1 to 30 % by weight and a water mobility value of less than 0.01 to a contact treatment with an oxidizing gas under fluidization, thereby keeping a temperature of the reaction mixture after the contact at 40°C or higher for 2 seconds or more. So far as some change is caused in the reaction mixture by the contact treatment with an oxidizing gas, the iron powder is not always required to be oxidized. However, it is preferable that the iron powder is oxidized. In that case, it is preferable that the iron powder becomes an active iron powder.

The "active heat generating composition" as referred to herein is a heat generating composition corresponding to any one of the following (1) to (3).

(1) A heat generating composition prepared by contact treating a reaction mixture containing, as essential components, an iron powder, a carbon component, a reaction accelerator and water with an oxidizing gas, or by adjusting the water content of the oxidizing gas contact treated mixture by the addition of water or a reaction accelerator aqueous solution.

(2) A heat generating composition prepared by contact treating a reaction mixture containing, as essential components, an iron powder, a carbon component, a reaction accelerator and water and having a water content of from 1 to 30 % by weight and a water mobility value of less than 0.01 with an oxidizing gas and holding the temperature of the reaction mixture at the time of contact at 40 °C or more for 2 seconds or more, or by subjecting the oxidizing gas and the contact treated mixture to adjustment of the water content by adjusting the water content of the oxidizing gas contact treated mixture by the addition of water or a reaction accelerator aqueous solution.

(3) A heat generating composition containing, as essential components, an iron powder, a carbon component, a reaction accelerator and water, wherein an iron powder containing from 20 to 100 % of an active iron powder is used as the iron powder.

The "moldable heat generating composition" as referred to herein is a heat generating composition which contains, as essential components, an iron powder, a carbon component, a reaction accelerator and water but does not contain a flocculant aid, a flocculant, an agglomeration aid, a dry binding material, a dry binding agent, a dry binder, a sticky raw material, a thickener and an excipient, contains surplus water so as to have a water mobility value of from 0.01 to 20 and has moldability due to the surplus water as a connecting substance, in which the water in the heat generating composition does not function as a barrier layer, the heat generating composition being capable of causing an exothermic reaction upon contact with air.

It is preferable that at least one of the foregoing packaging materials is made of a raw material preferably having a breaking strength of 400 g/mm$^2$ or more, more preferably 500 g/mm$^2$ or more, further preferably 1, 000 g/mm$^2$ or more, and still further preferably 2,000 g/mm$^2$ or more at 25°C and having a breaking elongation of 100 % or more at 90°C. Furthermore, though the thickness of the packaging material is not limited so far as the foregoing breaking elongation is secured, it is preferably 10 $\mu$m or more, more preferably from 10 to 500 $\mu$m, further preferably 10 to 300 $\mu$m, still further preferably from 10 to 250 $\mu$m, and even further preferably from 50 to 250 $\mu$m.

Laminates of a non-woven fabric and a film-like material of a thermoplastic resin are preferably enumerated.

At least one packaging material is a laminate of a fibrous material and a film-like material and is made of a raw material which is heat sealable and flexible. Furthermore, this packaging material at least has a breaking strength of 500 g/mm$^2$ or more under circumstances of from 25 to 60°C and a breaking elongation of 100 % or more at 90°C. In a heat generating body using this packaging material of the invention, the sectional exothermic parts containing a heat generating composition molded body or a heat generating composition compressed body as a compressed body of the heat generating composition molded body have a high bending resistance, and the sectioned parts as a heat seal part, which are present therebetween and do not contain a heat generating composition molded body or a heat generating composition compressed body (hereinafter referred to as "heat generating composition molded body") as a compressed body of the heat generating composition molded body have a low bending resistance. Since the exothermic part comprising a sectional exothermic part and a sectioned part can keep the bending resistance at a temperature of from about 0°C to about 80°C, the sectioned part functions as a hinge and is preferentially bent over the sectional exothermic part. In the heat generating body comprising a sectional exothermic part and a sectioned part, the sectioned part at least functions as a hinge at from the normal temperature to the temperature at the time of heating (from about 23°C to about 50°C) and is preferentially bent over the sectional exothermic part. A satisfactory difference of bending resistance at the time of heating is still kept. As a result, the heat generating body keeps structural support of the sectional exothermic part and has sufficient rigidity during the production or during the use. On the other hand, the excellent bending resistance at the time of heating is still kept.

In the heat generating body using the foregoing packaging material for at least one of the substrate or covering material, the heat generating composition molded body is laminated on a substantially planar substrate, a covering material is covered thereon, and the periphery of the heat generating composition molded body is heat sealed, thereby forming a sectioned part as a seal part. For example, in the case of using the foregoing packaging material for a covering material, since the packaging material is flexible and has a breaking strength of 500 g/mm$^2$ or more at 25°C at least at from 25 to 60°C, though it is bent, it has nerve and is able to surely cover the heat generating composition molded body. In addition, since the covering material has a breaking elongation of 100 % or more at 90°C at the time of heat seal, the covering material does not cause breakage by the temperature at the time of heat seal, is free from cutting in seal, and is able to surely form a heat seal part. Accordingly, in the heat generating body of the invention having an exothermic part comprising a sectional exothermic part containing a heat generating composition molded body and a sectioned part not containing a heat generating composition molded body, the sectional exothermic parts containing a heat generating composition molded body or a heat generating composition compressed body as a compressed body of the heat generating composition molded body have a high bending resistance, and the sectioned parts as a heat seal part, which are present therebetween and do not contain a heat generating composition molded body or a heat generating composition compressed body as a compressed body of the heat generating composition molded body have a low bending resistance. Since the exothermic part comprising a sectional exothermic part and a sectioned part can keep the bending resistance at a temperature of from about 0°C to about 80°C, the sectioned part functions as a hinge and is preferentially bent over the sectional exothermic part. Since the heat generating body comprising a sectional exothermic part and a sectioned part uses at least a packaging material which is small in change at the temperature of use, dimensional changes due to the packaging material are small at the time of use, and structural flexibility due to the bending resistance are kept. Thus, stable and appropriate flexibility is kept in the heat generating body. Furthermore, since in at least one of the both packaging materials, a packaging material (usually a covering material) having a breaking elongation of 100 % or more at 90°C is used, in the case of covering a substantially planar covering material on a heat generating composition molded body as laminated on a substantially planar substrate and heat sealing the periphery of the heat generating composition molded body, wrinkles which generate seal leakage are not generated so that a seal part free from cutting in seal can be formed. The covering material may partly have a convex.

In all of the foregoing working examples, the air-permeable covering materials as used were a non-elastic body having a permanent set of from 0. 5 % to 1. 7 % at a temperature between 25°C and 60°C and a laminate having a breaking strength of 400 g/mm$^2$ or more at 25°C and a breaking elongation of 20 % or more at 90°C.

The "seal strength at 60°C" as referred to herein means an average value of respective maximum values obtained by subjecting three samples to a measurement by taking a specimen of 25 mm × 250 mm from a place of a subjective sealed sample to be measured for the seal strength, allowing the specimen to stand under circumstances at 60°C for 5 minutes, grasping it under circumstances at 60°C, and measuring a maximum strength at intervals of 10 mm and at a tensile speed of 300 mm/min.

Here, the condition of the seal strength under circumstances at 20°C is identical with that of the seal strength at 60°C, except that the circumferential temperature for the measurement is 20°C.

The seal strength of the temporary adhering part is preferably 0.5 kg/25 mm or more, more preferably from 0.5 to 1 kg/25 mm, further preferably from 0.5 to 0.9 kg/25 mm, and still further preferably from 0.5 to 0.8 kg/25 mm under circumstances at 20°C; and the seal strength at 60°C is preferably less than 0.8 kg/25 mm, more preferably 0.01 kg/25 mm or more but less than 0.8 kg/25 mm, further preferably 0.01 kg/25 mm or more but less than 0.5 kg/25 mm, and still further preferably 0.01 kg/25 mm or more but less than 0.4 kg/25 mm.

The sticky layer of the temporary adhering part is constituted of an adhesive, has a seal strength at 60°C of from 0.01 to 0.8 kg/25 mm, is able to stop the movement of the heat generating composition molded body between the substrate and the covering material, and makes it possible to achieve heat sealing at high speed. In addition, if desired, warming may be carried out at the time of temporary adhesion. It is preferable that the warming is carried out under pressure at a temperature of not higher than a melting point of a base polymer in a hot melt based adhesive for forming the adhesive layer.

The seal strength under circumstances at 20°C of the heat seal part which has been heat sealed after the temporary adhesion is preferably 1.0 kg/25 mm or more, more preferably 1.2 kg/25 mm or more, further preferably 1.5 kg/25 mm or more, and still further preferably from 1.5 to 3 kg/25 mm. Furthermore, the seal strength at 60°C under circumstances at 60°C is preferably 0.8 kg/25 mm or more, more preferably 1.0 kg/25 mm or more, further preferably 1.2 kg/25 mm or more, and still further preferably 1.5 kg/25 mm or more.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable rawmaterials, and heat sealable rawmaterials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example. That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons); and examples of the layer H include non-water absorptive papers and water absorptive papers. Examples of the substrate or covering material include heat seal layer made of polyethylene obtained by using a metallocene catalyst/polypropylene film, polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/(paper and/or porous film)/perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10,000 $g/m^2/24$ hr, preferably from 70 to 5,000 $g/m^2/24$ hr, more preferably from 100 to 2, 000 $g/m^2/24$ hr, and further preferably from 100 to 700 $g/m^2/24$ hr in terms of moisture permeability by the Lyssy method.

When the moisture permeability is less 50 $g/m^2/24$ hr, the heat value is small and a sufficient thermal effect is not obtained, and therefore, such is not preferable. On the other hand, when it exceeds 10,000 $g/m^2/24$ hr, the exothermic temperature is high so that a problem in safety may possibly be generated, and therefore, such is not preferable. However, there is no limitation even when the moisture permeability exceeds 10,000 $g/m^2/24$ hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials,films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting fromholding a relatively large amount of a powder (for example, silica and alumina) on a synthetic resin (for example, polyethylene).

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectricsubstance, anantioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for j oint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-α-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an α-olefin. Also, polymers resulting from adding a diene (for example, butadiene and isoprene) as othermonomer theretomaybe employed.

The α-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodiumacrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1.0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar.

Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmicagents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm.

A capacity of the sectional exothermic part or a volume of the heat generating composition molded body is usually from 0.015 to 500 cm$^3$, preferably from 0.04 to 30 cm$^3$, more preferably from 0.1 to 30 cm$^3$, further preferably from 1 to 30 cm$^3$, and still further preferably from 3 to 20 cm$^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0.6 to 1, preferably from 0.7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.

Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.

Furthermore, the "volume of the heat generating composition molded body of the invention" as referred to herein means a volume of the heat generating composition molded body or compressed heat generating composition molded body. Furthermore, the "capacity of the sectional exothermic part" as referred to herein means an internal capacity of the sectional exothermic part having a heat generating composition molded body accommodated therein.

Further, the shape of the heat generating body is not limited but can be selected from the group consisting of a rectangular shape, a circular shape, an elliptical shape, a polygonal shape, a broad bean-like shape, an eye mask-like shape, a paper lantern-like shape, a cocoon-like shape, a gourd-like shape, a rectangular shape with rounded corners, a square shape with rounded corners, an egg-like shape, a boomerang-like shape, a comma-shaped bead-like shape, a wing-like shape, a nose-like shape, a star-like shape, and a foot-like shape.

Furthermore, the heat generating body or accommodating bag can be provided with at least one member of characters, designs, symbols, numerals, patterns, photographs, pictures, and colors in at least a part thereof.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.

Incidentally, examples of the pain of muscles or bones include acute muscle pain, acute bone pain, acute reference pain, previous muscle pain, previous bone pain, chronic reference pain, and join pain of knee, elbow, etc.

The holding time is not limited but is preferably from 20 seconds to 24 hours, more preferably from one hour to 24 hours, and further preferably from 8 hours to 24 hours.

The holding temperature is preferably from 30 to 50 °C, more preferably from 32 to 50 °C, further preferably from 32 to 43 °C, still further preferably from 32 to 41 °C, and even further preferably from 32 to 39 °C.

The working examples of the invention will be specifically described below on a basis of the drawings, but it should not be construed that the invention is limited thereto.

[Brief Description of the Drawings]

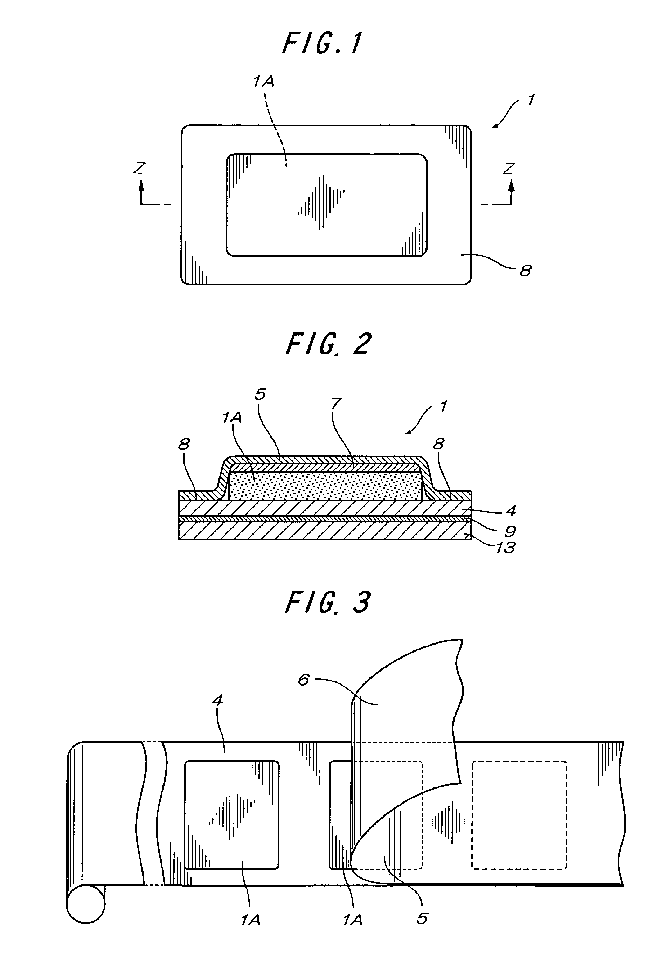

Fig. 1 is a plan view of an embodiment of a heat generating body of the invention.
Fig. 2 is a cross-sectional view along the line Z-Z of the same.
Fig. 3 is an oblique view to show the production step.

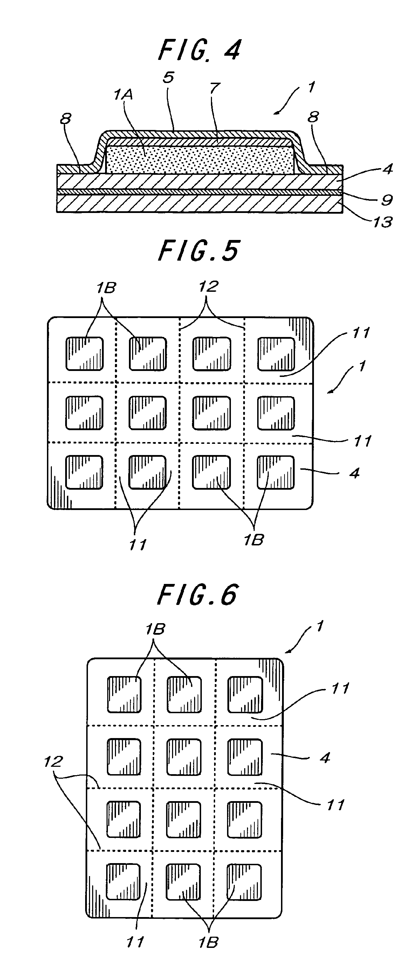

Fig. 4 is a cross-sectional view of an embodiment of the temporarily adhered heat generating body.
Fig. 5 is a plan view of another embodiment of the heat generating body of the invention.
Fig. 6 is a plan view of another embodiment of the heat generating body of the invention.
Fig. 7(a) is an oblique view of the temporary adhesion plate; and Fig. 7(b) is an oblique view of a modified example of the same.
Fig. 8(a) is an oblique view of an embodiment of the temporary adhesion roll; and Fig. 8(b) is a side view of the same.
Fig. 9(a) is an oblique view of another embodiment of the temporary adhesion roll; Fig. 9(b) is a cross-sectional view to show the relationship between the temporary adhesion roll and the heat generating body; Fig. 9(c) is a cross-sectional view to show the relationship between the temporary adhesion roll and the heat generating body; Fig. 9 (d) is an oblique view to show a part of the temporarily adhered heat generating body; and Fig. 9(e) is a cross-sectional view to show a part of the heat generating body partially having a sagging.

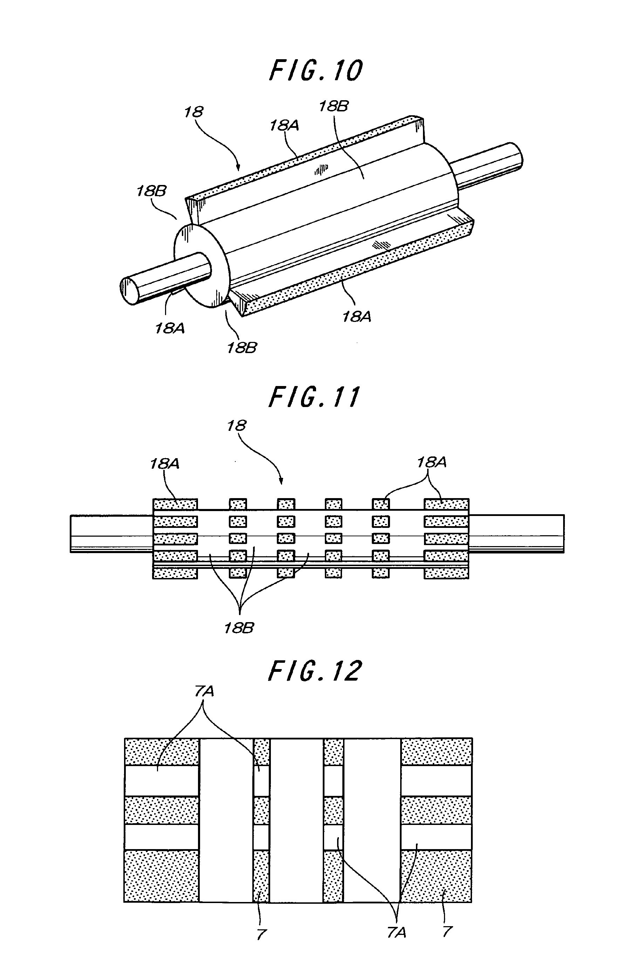

Fig. 10 is an oblique view of another embodiment of the temporary adhesion roll.
Fig. 11 is a plan view of another embodiment of the temporary adhesion roll.
Fig. 12 is a plan view of another embodiment of the temporary adhesion roll.
Fig. 13 is a plan view of another embodiment of the temporary adhesion roll.
Fig. 14 is a plan view of another embodiment of the temporary adhesion roll.
Fig. 15 is a side view of the collapsing plate of an embodiment of the sectional exothermic part of the heat generating body.
Fig. 16 is a side view of an embodiment of the collapsing roll of the sectional exothermic part of the heat generating body.

Figs. 17(a) and 17(b) are each a cross-sectional view of an embodiment of a part of the collapse step of the sectional exothermic part or exothermic part of the heat generating body.

Fig. 18 is a schematic view of an embodiment of the production step of the heat generating body.

Fig. 19 is a schematic view of another embodiment of the production step of the heat generating body.

Fig. 20 is a plan view of filter paper for the measurement of water mobility value in the invention.

Fig. 21 is an oblique view for explaining the measurement of water mobility value in the invention.

Fig. 22 is a side view for explaining the measurement of water mobility value in the invention.

Fig. 23 is a cross-sectional view for explaining the measurement of water mobility value in the invention.

Fig. 24 is a plan view of a filter paper after carrying out the measurement of water mobility value in the invention.

[Description of Reference Numerals and Signs]

| | |
|---|---|
| 1: | Heat generating body |
| 1A: | Heat generating composition molded body |
| 4: | Substrate |
| 5: | Covering material |
| 6: | Sagging |
| 7: | Temporary adhering part |
| 7B: | Space |
| 8: | Heat seal part (including a mixed layer of an adhesive and a heat sealing agent) |
| 9: | Adhesive layer |
| 9A: | Air-permeable adhesive layer |
| 11: | Sectioned part (seal part) |
| 12: | Perforation |
| 13: | Separator |
| 14: | Rotation body type molding device (body of rotation having a cavity having a desired shape) |
| 15: | Hopper |
| 17: | Temporary adhesion plate |
| 17A: | Temporary adhering seal part |
| 17B: | Concave |
| 18: | Temporary adhesion roll |
| 18A: | Temporary adhering seal part |
| 18B: | Concave |
| 19: | Heat seal roll |
| 20: | Cut roll |
| 21: | Delivery roll |
| 21A: | Press roll |
| 22: | Collapsing plate |
| 22A: | Collapsing part |
| 22B: | Collapsing roll |
| 22C: | Collapsing part |
| 22D: | Collapsing plate |
| 23: | Belt conveyor |
| 23A: | Belt conveyor |
| 24: | Melt blow machine |
| 25: | Melt blow machine |
| 26: | Pushing plate |
| 27: | Flat plate |
| 28: | Non-water absorptive film (polyethylene film, etc.) |
| 29: | Filter paper in which eight lines are drawn radiating from the central point with an interval of 45° |
| 30: | Die plate |
| 31: | Hole |
| 32: | Sample |
| 33: | Stainless steel plate |
| 34: | Distance to the oozed-out locus of water or aqueous solution |
| 35: | Position corresponding to a hollow cylindrical hole on filter paper (circumferential part) |

[Examples]

(Example 1)

As shown in Fig. 1 which is a plan view and Fig. 2 which is a cross-sectional view along the line Z-Z of Fig. 1, a heat generating body 1 of the invention is one in which a heat generating composition molded body 1A is sealed in a rectangular flat accommodating bag of 130 mm in length × 80 mm in width, and the accommodating bag is made of an air-impermeable substrate 4 and an air-permeable covering material 5. On the exposed surface side of the substrate 4, a separator 13 made of a polyester-made release film having a thickness of 38 $\mu$m is provided via an adhesive layer 9 which is made of a non-aromatic polyolefin based hot melt based adhesive having a basis weight of 150 g/m$^2$.

The covering material 5 is made of an extensible three-layered film having a width of 130 mm. That is, a porous film having a basis weight of 50 g/m$^2$ is laminated on a polyester-made spunlace non-woven fabric having a basis weight of 30 g/m$^2$ via a hot melt based sticky layer as provided by a melt blow system. This covering material 5 has a moisture permeability of 350 g/m$^2$/24 hr as measured by the Lyssy method.

The heat generating body 1 is produced as shown in Fig. 3.
While drawing out the heat generating body having the separator 13 provided on a polyethylene film-made substrate 4 via the adhesive layer 9 in a roll-like form at 30 m/min, the heat generating composition molded body 1A as molded in dimensions of 110 mm × 70 mm × 1. 7 mm was transferred at intervals of 20 mm into the central part in the side of the substrate 4 by means of force-through molding.
Next, as shown in Fig. 4, while drawing out the covering material 5 as wound in a roll-like form and applying an olefin based hot melt based adhesive at 1 g/m$^2$ onto the whole of the porous film surface side of the covering material 5 by a melt blow system, the heat generating composition molded body 1A was covered thereon such that its hot melt based adhesive layer 6 came into contact with the substrate 4, and the periphery thereof was contact bonded to form a temporary adhering part 7. Thereafter, the surroundings of the heat generating composition molded body 1A were heat sealed together with the temporary adhering part 8 by using a heat seal roll and then cut, thereby producing an extra-thin heat generating body of 8 mm in seal width × about 0.94 mm in thickness × 130 mm × 80 mm.
As a result of the production, seal failure was not observed.
Incidentally, each of the cut heat generating bodies 1 is subsequently sent to a packaging step and sealed in an airtight outer bag, an aspect of which is, however, not illustrated.

In this Example, a heat generating composition as prepared by adding water to 70 parts by weight of an iron powder (particle size: not more than 300 $\mu$m), 10 parts by weight of active carbon (particle size: not more than 300 $\mu$m), 2 parts by weight of salt, 0. 7 parts by weight of a water absorptive polymer (particle size: not more than 300 $\mu$m) and 0.1 parts by weight of calcium hydroxide so as to have a water mobility value of 8 was used as the moldable heat generating composition.
By regulating the water mobility value of the heat generating composition at 8 in this way, since it is not required to use a thickener, the exothermic characteristics are not sacrificed. Furthermore, it becomes easy to achieve lamination in the central part on the substrate by force-through molding; it is possible to achieve lamination with high precision in a desired laminated region; and it becomes possible to make the thickness of the heat generating composition molded body 1A very thin and uniformly control it.

Furthermore, the heat generating composition molded body was sealed in an outer bag; and after a lapse of 24 hours, the outer bag was broken, and the heat generating composition molded body was stuck on the surface of a human body and then provided for usual use. As a result, the temperature rose to about 38°C within about 1 to 2 minutes, and thereafter, the heat generation was continued at 38 to 41°C over 9 hours or more. The heat generating composition molded body did not move at all within the accommodating body during the use, and uniform heat generation was found over the entire surface.

(Example 2)

Fig. 5 is an example of the heat generating body 1 in which a sectional exothermic part 1B is provided by 3 lines and 4 rows in the same manner as in Example 1. A perforation 12 is provided in the heat seal part 11. Seal failure was not observed.

(Example 3)

Fig. 6 is an example of the heat generating body 1 having a sectional exothermic part 1B as produced by using the same substrate 4, covering material 5 and heat generating composition as in Example 1. The heat generating composition molded body 1B as formed in dimensions of 110 mm × 70 mm × 0.5 mm at intervals of 20 mm in the central part of a substrate 4 by force-through molding while drawing out the substrate 4 as wound in a roll-like form in a width of 130 mm at 35 m/min in the horizontal direction was disposed at intervals of 20 mm by 4 lines and 3 rows. A covering material as prepared by coating 1 g/m$^2$ of a hot melt based adhesive on the porous film surface side by a melt blow system was covered on the heat generating composition molded body 1B, and the outer periphery of the substrate 4 and the covering material and the adjacent sectional exothermic parts 1B were temporarily adhered by adhesion. Thereafter, the resultant was heat sealed by using three heat seal rolls and cut, thereby producing an extra-thin heat generating body of about 0. 94 mm in thickness $\times$ 130 mm $\times$ 80 mm. As a result, seal failure was not observed. Furthermore, the heat generating composition molded body was sealed in an outer bag; and after a lapse of 24 hours, the outer bag was broken, and the heat generating composition molded body was stuck on the surface of a human body and then provided for usual use. As a result, the same results as in Example 1 were obtained.

(Example 4)

A heat generating body of Example 4 is different in the points that while coating 1 g/m$^2$ of a hot melt based adhesive on a substrate and a heat generating composition molded body as formed in the same manner as in Example 1, a covering material was covered thereon such that the porous film side came into contact therewith. Incidentally, as the substrate, the covering material and the heat generating composition, the same materials as in Example 1 were used. A heat generating composition molded body of 5 mm in width $\times$ 110 mm in length $\times$ 3 mm in height was disposed by 4 lines and 2 rows (8 in total) at intervals of 7 mm on the substrate. The covering material was contact bonded in a width of 5 mm between the adjacent heat generating composition molded bodies, thereby providing a temporary adhering part. Next, the central part of the temporary adhering part was heat sealed in a width of 1 mm, and at the same time, the outer surroundings of the substrate and the covering material were heat sealed in a width of 8 mm, thereby obtaining a heat generating body having 8 sectional exothermic parts.

Next, the sectional exothermic parts were pressurized by using a planar deadhesion roll, thereby moving a part of the heat generating composition molded body into a non-heat sealed temporary adhering part, and the surroundings of the heat generating body was cut, thereby obtaining a heat generating body having a sectional exothermic part of 8 mm in seal width of the surroundings and 1 mm in seal width. Then, the heat generating body was sealed in an air-impermeable outer bag.

After sealing the heat generating composition molded body in an outer bag and then lapsing 24 hours, the outer bag was broken, and the heat generating composition molded body was stuck on the surface of a human body and then provided for usual use. As a result, the temperature rose to about 38°C within about 1 to 2 minutes, and thereafter, the heat generation was continued at 38 to 41°C over 10 hours ormore. The heat generating composition molded body did not move at all within the accommodating body during the use and was flexible as a heat generating body and well adaptive with the curved surfaces of the body. Uniform heat generation was found over the entire surface.

(Example 5)

Fig. 18 is a schematic view of the production step of a heat generating body including a temporary adhering step. A heat generating composition 2 is charged in a hopper 15 while drawing out a substrate 4 at 15 m/min from a delivery roll 21. Then, a heat generating composition molded body of 110 mm $\times$ 70 mm is formed at intervals of 20 mm in the central part on the substrate 4 on a belt conveyor 23 from the hopper 15 via a rotation body type molding device 14. Incidentally, the heat generating composition 2 is attracted by a magnet 37 provided beneath an upper belt of the belt conveyor 23 so that it is easily placed on the substrate 4.

In addition, the covering material 5 is covered on the substrate 4 and the heat generating composition molded body by temporary adhesion rolls 18 in the MD direction which also functions as a doubling calender during traveling on the belt conveyor 23. A hot melt based adhesive is coated on the entire surface of the covering material 5 in the porous film side by a melt blow machine 24.

Next, the periphery of the heat generating composition molded body was heat sealed in a width of 5 mm, and at the same time, the outer periphery of the heat generating body was heat sealed in a width of 8 mm by heat seal rolls 18C. Next, the heat generating composition molded body was cut by using die cut rolls 20, thereby obtaining a heat generating body of 126 mm $\times$ 86 mm. In the resulting heat generating body, heat seal failure was not observed.

The cut heat generating body was subsequently sent to a packaging step and sealed in an airtight outer bag (as not illustrated). After sealing the heat generating composition molded body in an outer bag and then lapsing 24 hours, the outer bag was broken, and the heat generating composition molded body was stuck on the surface of a human body and then provided for use. As a result, the same results as in Example 1 were obtained.

In this Example, a measure for providing an air-permeable adhesive layer is not particularly limited so far as an air-permeable adhesive layer can be formed. A melt blow method, a curtain rail method, and the like can be employed. Furthermore, a heat generating composition consisting of 70 parts by weight of an iron powder (particle size: not more than 300 $\mu$), 7 parts by weight of active carbon (particle size: not more than 300 $\mu$), 2 parts by weight of salt, 0.7 parts by weight of a water absorptive polymer, 3.0 parts by weight of a wood meal, 1.0 part by weight of sodium sulfite, 0.1 parts by weight of calcium hydroxide, and 37 parts by weight of water was used as the heat generating composition of this Example.

In Fig. 19, a step for reinforcing the heat seal step as shown in Fig. 18 and providing a separator-provided air-permeable adhesive layer is established. In the established step, the heat generating composition molded body other than an air-permeable adhesive layer 9A as coated with a hot melt adhesive on a silicone-treated polyester-made separator 13 by a melt blow system was laminated on a covering material 5 and cut by die cut rolls 20.

(Example 6)

In this Example, a heat generating body was produced in the same manner as in Example 5.

As the heat generating composition of this Example, a reaction mixture consisting of 100 parts by weight of an iron powder (particle size: not more than 300 $\mu$m), 6. 5 parts by weight of active carbon (particle size: not more than 300 $\mu$m), 2.3 parts by weight of a wood meal (particle size: not more than 150 $\mu$m), 2.3 parts by weight of a water absorptive polymer (particle size: not more than 300 $\mu$m), 0.5 parts by weight of calcium hydroxide, 0.7 parts by weight of sodium sulfite and 10 parts by weight of 6 % salt water and having a water mobility value of less than 0.01 was charged in a stirring type batchwise oxidizing gas contact treatment device. In the state that the upper portion of the oxidizing gas contact treatment device was opened to air, the reaction mixture was stirred under circumstances at 20°C. After 120 seconds, at the point of time when a temperature rise of the reaction mixture reached 45°C, the reaction mixture was sealed in an air-impermeable accommodating bag and cooled to room temperature to obtain a heat generating mixture of the invention. Next, 6 % salt water was added and mixed in the heat generating mixture, thereby obtaining a heat generating composition having a water mobility value of 10, which was then used as the heat generating composition of the invention.

**Claims**

1.  A heat generating body as formed by laminating a heat generating composition molded body resulting from molding of a heat generating composition containing, as essential components, an exothermic substance, a carbon component, a reaction accelerator and water and having a water mobility value of from 0.01 to 20 on a substrate, covering by a covering material and heat sealing the periphery of the heat generating composition molded body, **characterized in that**:

    the substrate and the covering material are temporarily adhered with a sticky layer, the temporarily adhered portion is heat sealed with a heat seal layer which the substrate and/or the covering material has, the sticky layer component and the heat seal layer component are co-present in the heat seal part, and the heat seal part has a seal strength at 60°C of 0.8 kg/25 mm or more.

2.  The heat generating body according to claim 1, **characterized in that** at least a part of the heat seal part has a region having an air vent part heat sealed therein.

3.  The heat generating body according to claim 1, **characterized in that**, the sticky layer is constituted of a hot melt based adhesive.

4.  The heat generating body according to claim 1, **characterized in that** the total thickness of the heat seal layer is the thickness of the sticky layer or more.

5.  The heat generating body according to claim 1, **characterized in that** the sticky layer has a thickness of from 0.1 to 100 $\mu$m.

6.  The heat generating body according to claim 1, **characterized in that** the sticky layer has a void.

7.  The heat generating body according to claim 1, **characterized in that** a mixed part of the heat seal layer component and the adhesive layer component is present in the heat seal part.

8.  The heat generating body according to claim 1, **characterized in that** the substrate and the covering material are formed of an extensible raw material.

9. The heat generating body according to claim 1, **characterized in that** the heat generating composition molded body is sectioned by a sectioned part which is a heat seal part and is disposed in a plural number.

10. The heat generating body according to claim 1, **characterized in that** the exothermic substance in the heat generating composition comprising iron powder particles, and the iron powder particles have iron oxide film.

11. The heat generating body according to claim 1, **characterized in that** the heat generating composition molded body is compression treated.

12. The heat generating body according to claim 1, **characterized in that** after heat sealing, at least a part of the heat generating composition molded body is moved into the temporary adhering part which is not heat sealed, thereby deadhering the temporary adhering part which is not heat sealed.

13. The heat generating body according to claim 1, **characterized in that** the heat generating body has a fixing measure on the exposed surface thereof.

14. A process for producing a heat generating body, **characterized in that** an air-permeable substrate and/or covering material at least has a heat sealable heat seal layer; a heat generating compositionmoldedbody resulting frommolding of a heat generating composition containing, as essential components, an iron powder, a carbon component, a reaction accelerator and water and having a water mobility value of from 0.01 to 20 is laminated on the substrate; a hot melt based adhesive is provided as a sticky layer thereon by a melt blow system; the covering material is covered thereon; the heat generating composition molded body and/or the substrate is temporarily adhered to the covering material by temporary adhesion rolls; the periphery of the heat generating composition molded body is then heat sealed; and the seal part has a seal strength at 60°C of 0.8 kg/25 mm or more.

15. The process for producing a heat generating body as according to claim 14, **characterized in that** after temporarily adhering the outer circumference of the heat generating body, the outer circumstance of the heat generating body is heat sealed while retaining an air vent part.

16. The process for producing a heat generating body according to claim 14, **characterized in that** heat sealing is achieved by using heat rolls having the heat seal part smaller than the temporary adhering part in the temporary adhesion rolls.

17. The process for producing a heat generating body according to claim 14, **characterized in that** the heat generating composition molded body is moved into the temporary adhering part which is not heat sealed, thereby deadhering the temporary adhering part which is not heat sealed.

## FIG. 1

## FIG. 2

## FIG. 3

## FIG. 4

## FIG.5

## FIG.6

FIG.7(a)

FIG.7(b)

FIG.8(a)

FIG.8(b)

FIG.9(a)

FIG.9(b)

FIG.9(c)

FIG.9(d)

FIG.9(e)

## FIG.10

## FIG.11

## FIG.12

## F I G . 1 3

## F I G . 1 4

## F I G . 1 5

## F I G . 1 6

## Fig. 17(a)

## Fig. 17(b)

## Fig. 18

## FIG.19

## FIG.20

## FIG.21

## FIG.22

## FIG.23

## FIG.24

# EP 1 782 774 A1

<table>
<tr><td colspan="2">INTERNATIONAL SEARCH REPORT</td><td>International application No.<br>PCT/JP2005/013003</td></tr>
</table>

A. CLASSIFICATION OF SUBJECT MATTER
    Int.Cl⁷ A61F7/08, C09K5/16

According to International Patent Classification (IPC) or to both national classification and IPC

B. FIELDS SEARCHED

Minimum documentation searched (classification system followed by classification symbols)
    Int.Cl⁷ A61F7/08, C09K5/16

Documentation searched other than minimum documentation to the extent that such documents are included in the fields searched
    Jitsuyo Shinan Koho          1922-1996    Jitsuyo Shinan Toroku Koho    1996-2005
    Kokai Jitsuyo Shinan Koho    1971-2005    Toroku Jitsuyo Shinan Koho    1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2002-155273 A (Kaoru USUI),<br>28 May, 2002 (28.05.02),<br>Full text; Figs. 1 to 14<br>(Family: none) | 1-8,10,11,<br>13,14-16 |
| A | JP 2003-336042 A (Maikoru Kabushiki Kaisha),<br>28 November, 2003 (28.11.03),<br>Full text; Figs. 1 to 16<br>& US 2004/0217325 A        & EP 1516900 A<br>& WO 2003/097764 A1        & CA 2439044 A<br>& CN 1496395 A | 1-8,10,11,<br>13,14-16 |

[X] Further documents are listed in the continuation of Box C.          [ ] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    14 September, 2005 (14.09.05) | Date of mailing of the international search report<br>    04 October, 2005 (04.10.05) |
|---|---|
| Name and mailing address of the ISA/<br>    Japanese Patent Office | Authorized officer |
| Facsimile No. | Telephone No. |

Form PCT/ISA/210 (second sheet) (January 2004)

**INTERNATIONAL SEARCH REPORT**

| International application No. |
| --- |
| PCT/JP2005/013003 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2003-129041 A (Maikoru Kabushiki Kaisha), 08 May, 2003 (08.05.03), Column 48, line 31 to column 49, line 37; Figs. 1, 5, 7 to 9, 13 & US 2004/42965 A1 & EP 1439213 A1 & WO 2003/035792 A1 & CA 2432544 A & CN 1491271 A | 1-8,10,11, 13,14-16 |
| Y | JP 2002-36471 A (Nitto Denko Corp.), 05 February, 2002 (05.02.02), Column 2, line 25 to column 3, line 36; column 8, lines 2 to 19; table 1 (Family: none) | 1 |
| Y | JP 2004-24671 A (Maikoru Kabushiki Kaisha), 29 January, 2004 (29.01.04), Page 9, lines 9 to 14 (Family: none) | 2,15 |
| Y | JP 3007467 U (Kiribai Kagaku Kabushiki Kaisha), 14 February, 1995 (14.02.95), Page 9, line 17 to page 10, line 14; Figs. 1 to 8 (Family: none) | 9 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60003350 B
- JP 62087267 A
- JP 63017295 B
- JP 1059023 A
- JP 2001507593 T
- JP 3161605 A
- JP 11508314 T
- JP 3161605 B

- JP 2002200108 A
- JP 10265373 A
- JP 9087173 A
- JP 6145050 A
- JP 6199660 A
- JP 10279466 A
- JP 10182408 A